# EUROPEAN PATENT SPECIFICATION

(11) **EP 3 160 511 B1**
(45) Date of publication and mention of the grant of the patent: **26.07.2023**
(21) Application number: 15738505.5
(22) Date of filing: 15.06.2015
(51) Int. Cl.: A61K 38/18, A61K 47/20, A61P 7/06, A61P 37/02, A61K 47/26, A61K 9/19, A61K 47/18

(54) **PROTEIN FORMULATIONS**
PROTEINFORMULIERUNGEN
FORMULATIONS DE PROTÉINES

(30) Priority: 26.06.2014 US 201462017560 P
(43) Date of publication of application: 03.05.2017
(73) Proprietor: Amgen Inc., Thousand Oaks, California 91320-1799 (US)
(72) Inventor: PARK, Sungae, Thousand Oaks, California 91320 (US); MCAULEY, Arnold J., Moorpark, California 93021 (US)
(74) Representative: Dörries, Hans Ulrich
(86) International application number: PCT/US2015/035884
(87) International publication number: WO 2015/200027

(56) References cited:
- WO-A1-2010/148337
- WO-A2-03/009817
- WO-A2-2007/014073
- WO-A2-2011/035335
- US-A1- 2012 076 800

## Description

This application claims the benefit of U.S. Provisional Application Serial Number 62/017,560 filed June 26, 2014.

### FIELD OF THE INVENTION

The field of this invention relates to compositions and methods related to protein formulations.

### BACKGROUND OF THE INVENTION

Therapeutic protein drugs can be unstable and vulnerable to environmental and other external influences, potentially leading to changes that reduce their activity. Attempts to formulate therapeutic proteins can frequently lead to formulations that cause patient discomfort (*e.g.*, low pH formulations).

In some instances, therapeutic protein formulations are stabilized by adding by adding additional proteins to the formulation. For example, human serum albumin (HSA) has been used as a stabilizer. However, the addition of additional proteins to a pharmaceutical product is not ideal for a variety of reasons, including the risk of adverse immune response or viral contamination.

WO 2007/014073 discloses a solution comprising a protein, like e.g. darbepoietin alpha, at approximately 40 to 150 mg/ml, approximately 7 to 50 mM histidine, approximately 2% to 4% mannitol, approximately 1.0 to 2.5% sucrose, and approximately 0.004% to 0.015% polysorbate 20 or polysorbate 80, with pH 4.5 to 7.5.

Accordingly, there is a need for protein formulations that provide long term stability at various temperatures and that minimize pain associated with injection.

### SUMMARY OF THE INVENTION

In one embodiment, the invention provides a solid protein formulation comprising a stabilizer, a sugar alcohol, a sugar and a surfactant, as defined by the claims.

In another embodiment, the invention provides a solid protein formulation, comprising darbepoetin alfa, histidine, mannitol, sucrose, and polysorbate-80. Also disclosed, but not according to the invention is a solid protein formulation comprising sodium glutamate, mannitol, sucrose, and polysorbate 20. Also disclosed, but not according to the invention is a solid protein formulation comprising histidine, mannitol, sucrose, and polysorbate 20. In a further embodiment, the invention provides a method of preparing the solid protein formulation comprising: a) diluting said protein with a lyophilization buffer; and b) lyophilizing said diluted protein. In another embodiment, the invention provides a kit comprising solid protein formulations and a reconstitution buffer.

### BRIEF DESCRIPTION OF THE FIGURES

**Figure 1** describes the results of a comparison of denaturation of Aranesp using the detecting antibody 9G8A without (A) and with (B) polysorbate-80 at 37 °C.

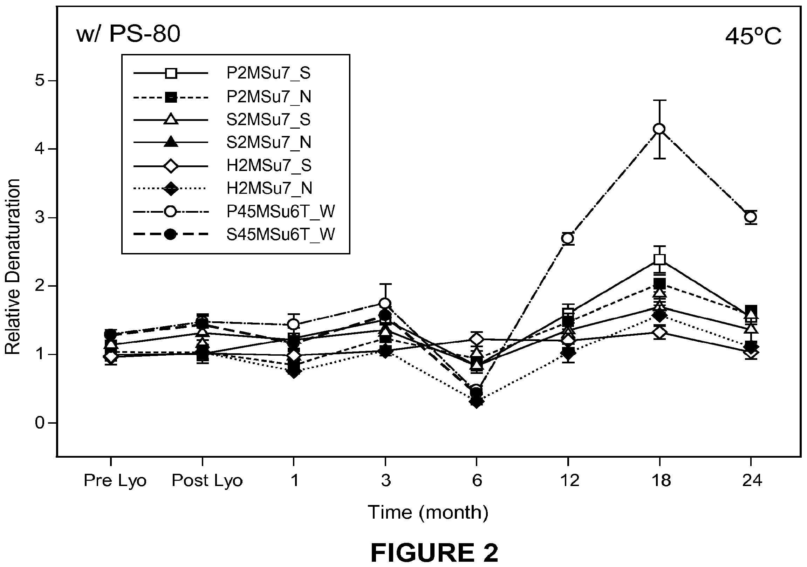

**Figure 2** describes the results of a comparison of denaturation of Aranesp using the detecting antibody 9G8A with polysorbate-80 at 45 °C.
**Figure 3** describes the results of a comparison of % main peak by SEC of Aranesp^{®} without polysorbate-80 in lyophilized formulation compared to liquid formulation at 37 °C and 45 °C.
**Figure 4** describes an SEC-HPLC overlay of samples not containing Polysorbate-80 reconstituted with 4% Sorbitol at 24months at 45 °C. (A) Represents overlay of samples zoomed, and (B) represents overlays of samples unzoomed.
**Figure 5** describes the results of a comparison of % main peak by SEC of Aranesp^{®} without Polysorbate-80 among lyophilized formulation at 45 °C.
**Figure 6** describes the results of a comparison of % main peak by SEC of Aranesp^{®} with polysorbate-80 among lyophilized formulation at 45 °C.

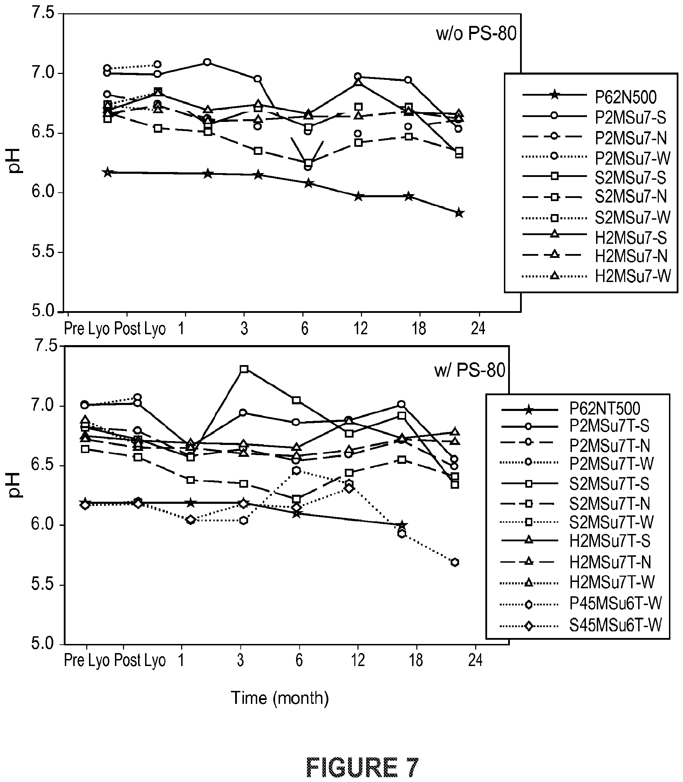

**Figure 7** describes the results of a comparison of pH of Aranesp^{®} with and without polysorbate-80 among lyophilized and liquid formulation at 45 °C.
**Figure 8** describes the results of a comparison of Aranesp % oxidation after storage at 4 °C for up to 24 months.
**Figure 9** describes the results of a comparison of % oxidation of Aranesp in liquid and Lyo formulation after storage at 29 °C for up to 24 months: P62N500 and P62N500T are liquid formulation and the other samples are a lyophilized formulation.
**Figure 10** describes the results of a comparison % oxidation of Aranesp in lyo formulation only after storage at 29 °C.
**Figure 11** describes the results of a comparison % oxidation of Aranesp in liquid and Lyo formulation after storage at 37 °C for up to 24 months: P62N500 and P62N500T are liquid formulation and rests of them are lyophilized formulation.
**Figure 12** describes the results of a comparison of % oxidation of Aranesp in Lyo formulation only after storage at 37 °C.
**Figure 13** describes the results of a comparison % oxidation of Aranesp in liquid and Lyo formulation after storage at 45 °C for up to 24 months: P62N500 and P62N500T are liquid formulation and the remaining samples are lyophilized formulation.

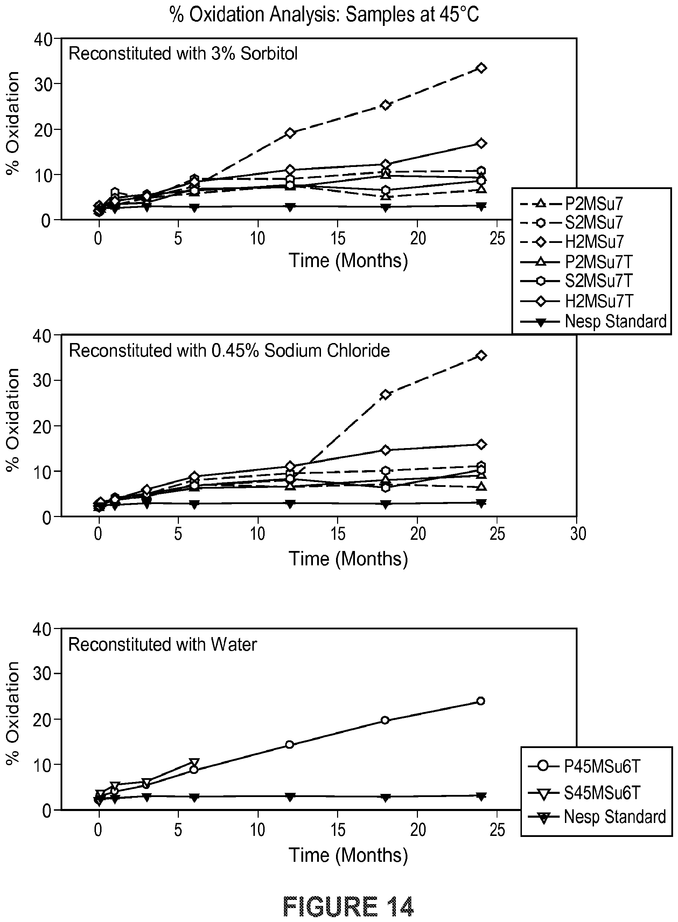

**Figure 14** describes the results of a comparison % oxidation of Aranesp in Lyo formulation only after storage at 37 °C for up to 24 months.

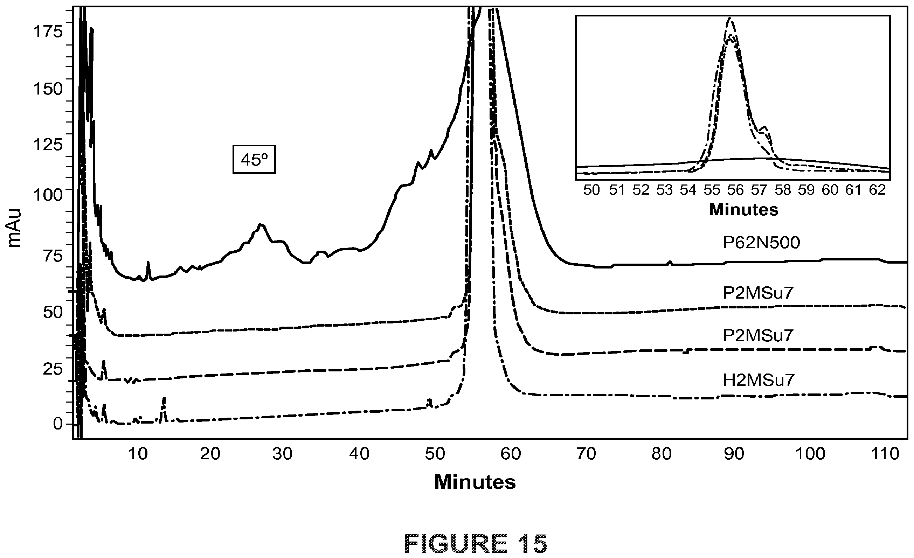

**Figure 15** describes the results of a comparison of Aranesp (500 µg/mL) without polysorbate-80 in three lyophilized and reconstituted in 4 % Sorbitol between Aranesp liquid formulation for degradation and clip species by non-reduced reverse phase HPLC after being stored for 24 month at 45 °C.

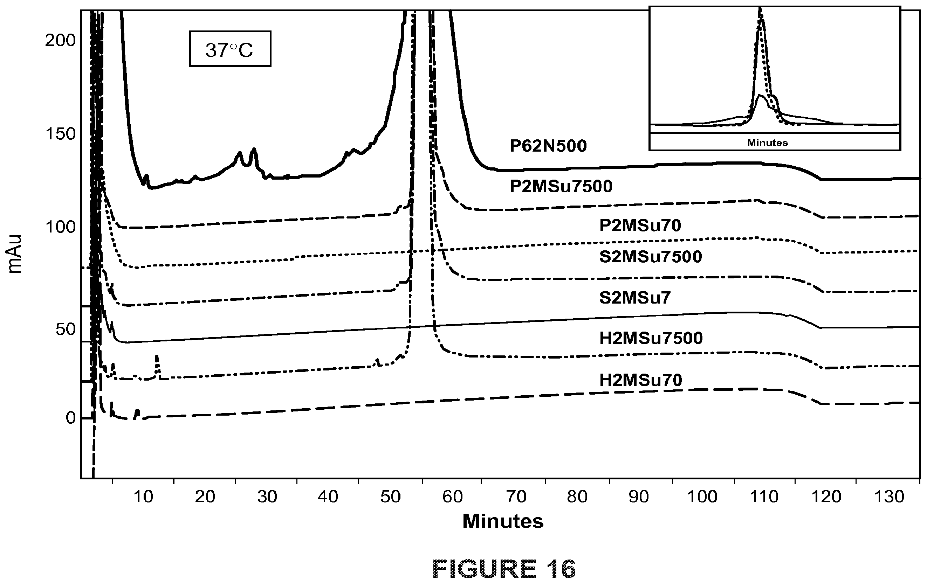

**Figure 16** describes the results of a comparison of Aranesp without polysorbate-80 in three lyophilized and reconstituted in 4 % Sorbitol between Aranesp liquid formulation for degradation and clip species by non-reduced reverse phase HPLC after stored for 24 month at 37 °C.

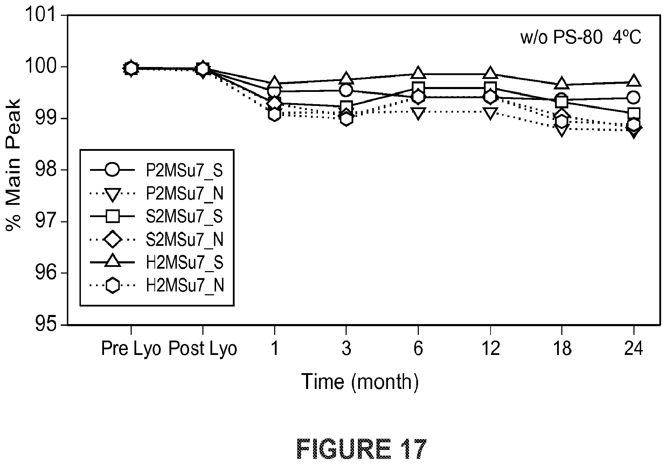

**Figure 17** describes SEC-HPLC % main peak results of samples not containing polysorbate 80 at 4°C.

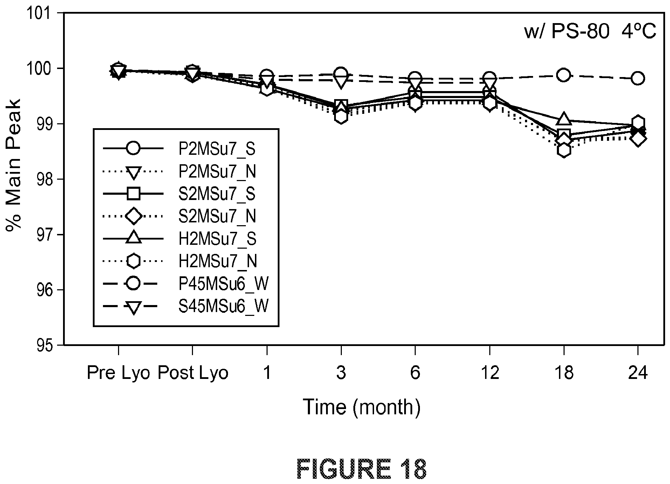

**Figure 18** describes SEC-HPLC % Main peak results of samples containing polysorbate 80 at 4°C.

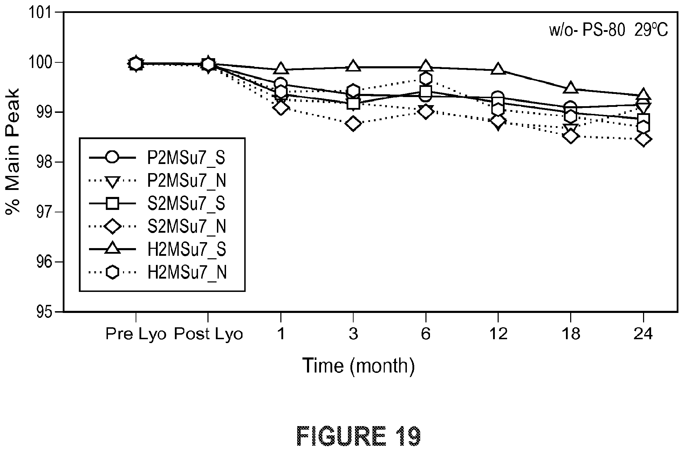

**Figure 19** describes SEC-HPLC % Main peak results of samples not containing polysorbate 80 at 29°C.

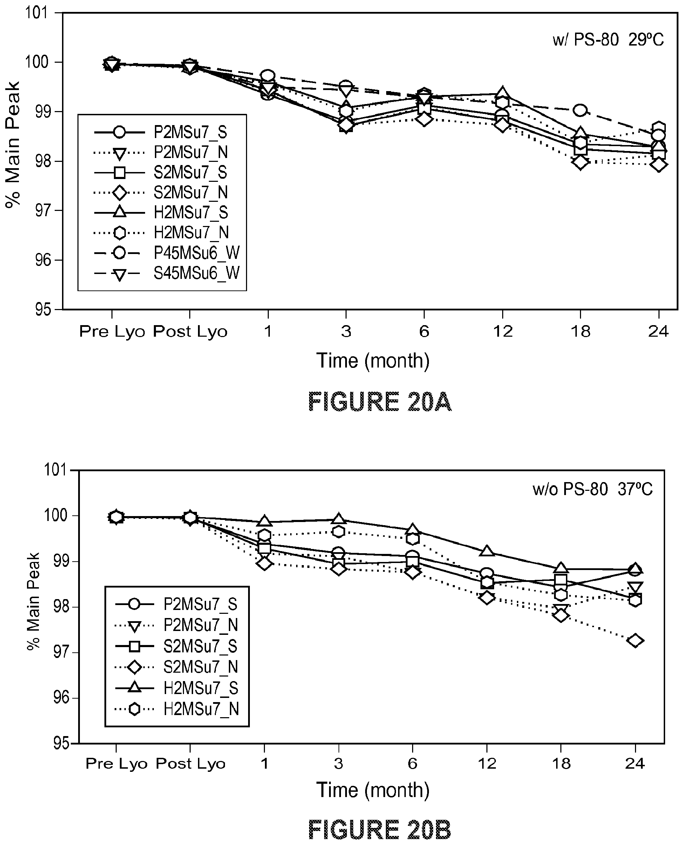

**Figure 20A** describes SEC-HPLC % Main peak results of samples containing polysorbate 80 at 29°C. **Figure 20B** describes SEC-HPLC % Main peak results of samples not containing polysorbate 80 at 29°C.

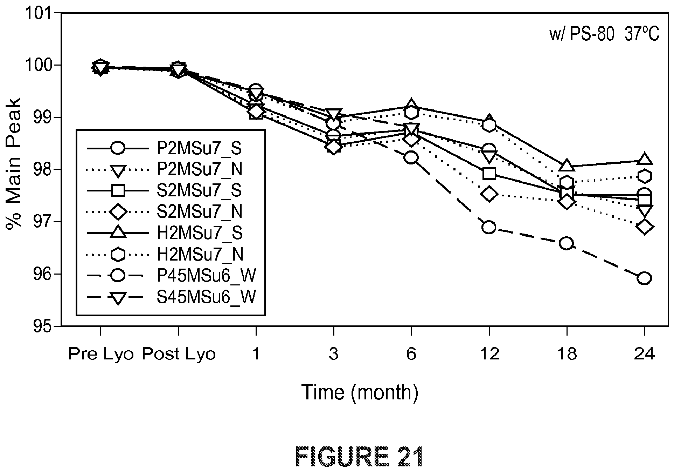

**Figure 21** describes SEC-HPLC % Main peak results of samples containing polysorbate 80 at 37°C.
**Figure 22** describes the results of a comparison of Aranesp^{®} 9G8A results at different temperatures between CZ and glass vial. CZ container showed higher denaturation at higher temperature (25 °C, 37 °C, 45 °C).
**Figure 23** describes the results of a comparison of % HMW by SEC of Aranesp^{®} in two different containers with three different reconstitution diluents at different temperatures.
**Figure 24** describes the results of a comparison of % main peak by SEC of Aranesp^{®} in two different containers with three different reconstitution diluents at different temperatures.
**Figure 25** describes the results of a HIAC particle count per mL results of protein samples at different temperatures.
**Figure 26** describes visual observation results of lyophilized samples. At 12 months, samples showed low amount of lyophilized cake in CZ vial at 4 °C. Vials with blue caps contain protein samples. Vials with green caps contain placebo samples.

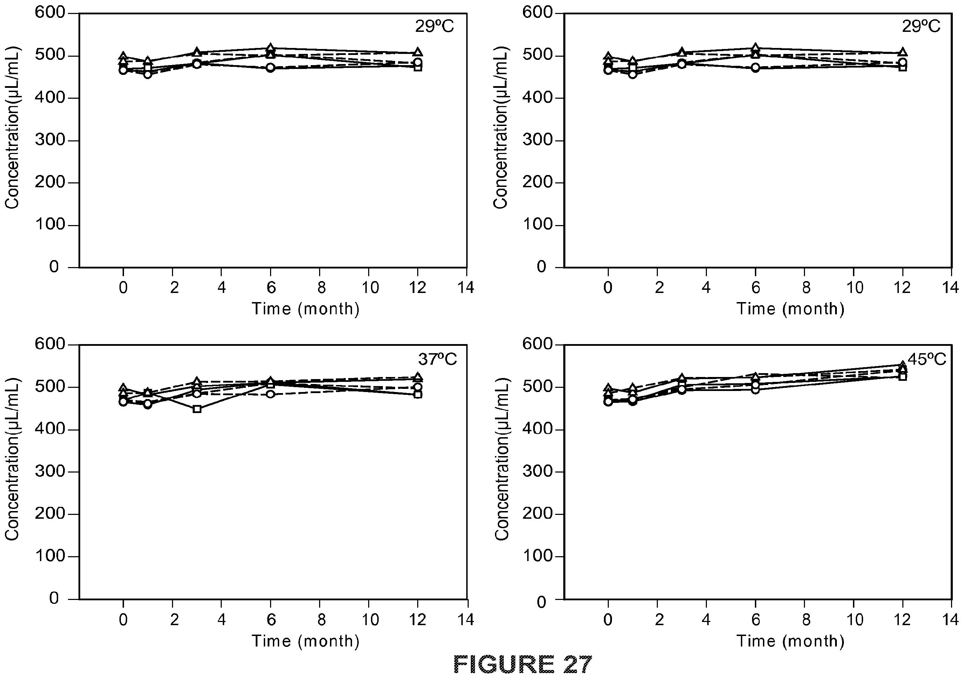

**Figure 27** describes the results of a comparison of Aranesp^{®} concentration (µg/mL) at different temperatures.
**Figure 28** describes the results of a comparison of % oxidation of Aranesp after stored at four different temperatures (4 °C, 29 °C, 37 °C and 45 °C) in two different containers (CZ and Glass vial).
**Figure 29** describes the results of a comparison of clip species of Aranesp by non-reduced reverse phase HPLC after stored for 12 month at four different temperatures in two different containers.
**Figure 30** describes the results of a comparison of pH of Aranesp at four different temperatures (4 °C, 29 °C, 37 °C and 45 °C) in two different containers (CZ and Glass vial).
**Figure 31** describes the results of a comparison of osmolarity of Aranesp at four different temperatures (4°C, 29°C, 37°C and 45°C) in two different containers (CZ and Glass vial).
**Figure 32** describes the results of % low molecular weight analysis of sample by SEC at 4°C and 37°C up to 12 weeks for 100 µg/mL and 500 µg/mL sample at two different lyophilization formulations.
**Figure 33** describes the results of % high molecular weight analysis of sample by SEC at 4°C and 37°C up to 12 weeks for 100 µg/mL and 500 µg/mL sample at two different lyophilization formulations.
**Figure 34** describes the results of % main peak of sample analysis by SEC at 4°C and 37°C up to 12 weeks for 100 µg/mL and 500 µg/mL sample at two different lyophilization formulations.
**Figure 35** describes the results of % heavy chain analysis of sample by CE-SDS at 4°C and 37°C up to 12 weeks for 100 µg/mL and 500 µg/mL sample at two different lyophilization formulations.
**Figure 36** describes the results of % light chain analysis of sample by CE-SDS at 4°C and 37°C up to 12 weeks for 100 µg/mL and 500µg/mL sample at two different lyophilization formulations.
**Figure 37** describes the results of % non-glycosylated heavy chain analysis of sample by CE-SDS at 4°C and 37°C up to 12 weeks for 100µg/mL and 500 µg/mL sample at two different lyophilization formulations
**Figure 38** describes the results of an electropherogram of EPO mAb by CE-SDS after stored 7 weeks at 4°C and 37°C for 500 µg/mL sample at two different lyophilization formulations: H= Histidine formulation, G = Glutamate formulation.
**Figure 39** describes the results of DSC scans of the pre-lyo samples in GMST and HMST buffers. Black = 0.1 mg/mL anti-EPO mAb 8C10-GMST, red = 0.1 mg/mL anti-EPO mAb 8C10-HMST, green = 0.5 mg/mL anti-EPO mAb 8C10-GMST, and blue = 0.5 mg/mL anti-EPO mAb 8C10-HMST. The signals of the 0.1 mg/mL samples were normalized to 0.5 mg/mL (the same normalization was used for all of the 0.1 mg/mL samples).
**Figure 40** describes the results of DSC scans of the 0.1 mg/mL anti-EPO mAb 8C10-GMST samples under different conditions. Black = pre-lyo, red = lyo (T=0), green = lyo (T=7 weeks at 4°C), blue = lyo (T=7 weeks at 37°C), cyan = lyo (T=12 weeks at 4°C), and dark yellow = lyo (T=12 weeks at 37°C).
**Figure 41** describes the results of DSC scans of the 0.1 mg/mL anti-EPO mAb 8C10-HMST samples under different conditions. Black = pre-lyo, red = lyo (T=0), green = lyo (T=7 weeks at 4°C), and blue = lyo (T=7 weeks at 37°C).
**Figure 42** describes the results of DSC scans of the 0.5 mg/mL anti-EPO mAb 8C10-GMST samples under different conditions. Black = pre-lyo, red = lyo (T=0), green = lyo (T=7 weeks at 4°C), blue = lyo (T=7 weeks at 37°C), cyan = lyo (T=12 weeks at 4°C), and dark yellow = lyo (T=12 weeks at 37°C).
**Figure 43** describes the results of DSC scans of the 0.5 mg/mL anti-EPO mAb 8C10-HMST samples under different conditions. Black = pre-lyo, red = lyo (T=0), green = lyo (T=7 weeks at 4°C), and blue = lyo (T=7 weeks at 37°C).
**Figure 44** describes the results of size distribution analysis of anti EPO mAb samples at T=0 (magnified 10×).
**Figure 45** describes the results of AUC analyses of anti EPO mAb samples. Glutamate buffers are upper panel, histidine buffers lower panels; 4 °C are presented blue and 37 °C red. PreLyo samples are T=0 in all graphs.

### DETAILED DESCRIPTION OF THE INVENTION

The present invention relates to protein formulations, as defined by the claims. The present invention further provides compositions, kits, and methods relating to protein formulations, as defined by the claims.

### Definitions

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. Generally, nomenclatures used in connection with, and techniques of, cell and tissue culture, molecular biology, immunology, microbiology, genetics and protein and nucleic acid chemistry and hybridization described herein are those well known and commonly used in the art. The methods and techniques of the present invention are generally performed according to conventional methods well known in the art and as described in various general and more specific references that are cited and discussed throughout the present specification unless otherwise indicated. See, e.g., Sambrook et al. Molecular Cloning: A Laboratory Manual, 2d ed., Cold Spring Harbor Laboratory Press, Cold Spring Harbor, N.Y. (1989) and Ausubel et al., Current Protocols in Molecular Biology, Greene Publishing Associates (1992), and Harlow and Lane Antibodies: A Laboratory Manual Cold Spring Harbor Laboratory Press, Cold Spring Harbor, N.Y (1990). Enzymatic reactions and purification techniques are performed according to manufacturer's specifications, as commonly accomplished in the art or as described herein. The terminology used in connection with, and the laboratory procedures and techniques of, analytical chemistry, synthetic organic chemistry, and medicinal and pharmaceutical chemistry described herein are those well known and commonly used in the art. Standard techniques can be used for chemical syntheses, chemical analyses, pharmaceutical preparation, formulation, and delivery, and treatment of patients.

The following terms, unless otherwise indicated, shall be understood to have the following meanings:
A "glycoprotein" is a protein that has covalently attached oligosaccharides to polypeptide side chains.

An "immunoglobulin" is a tetrameric molecule. In a naturally occurring immunoglobulin, each tetramer is composed of two identical pairs of polypeptide chains, each pair having one "light" (about 25 kDa) and one "heavy" chain (about 50-70 kDa). The amino-terminal portion of each chain includes a variable region of about 100 to 110 or more amino acids primarily responsible for antigen recognition. The carboxy-terminal portion of each chain defines a constant region primarily responsible for effector function. Human light chains are classified as kappa and lambda light chains. Heavy chains are classified as mu, delta, gamma, alpha, or epsilon, and define the antibody's isotype as IgM, IgD, IgG, IgA, and IgE, respectively. Within light and heavy chains, the variable and constant regions are joined by a "J" region of about 12 or more amino acids, with the heavy chain also including a "D" region of about 10 more amino acids. See generally, Fundamental Immunology Ch. 7 (Paul, W., ed., 2nd ed. Raven Press, N.Y. (1989)). The variable regions of each light/heavy chain pair form the antibody binding site such that an intact immunoglobulin has two binding sites.

An "antibody" refers to an intact immunoglobulin or to an antigen binding portion thereof that competes with the intact antibody for specific binding, unless otherwise specified. Antigen binding portions may be produced by recombinant DNA techniques or by enzymatic or chemical cleavage of intact antibodies. Antigen binding portions include, inter alia, Fab, Fab', F(ab')₂, Fv, domain antibodies (dAbs), fragments including complementarity determining regions (CDRs), single-chain antibodies (scFv), chimeric antibodies, diabodies, triabodies, tetrabodies, and polypeptides that contain at least a portion of an immunoglobulin that is sufficient to confer specific antigen binding to the polypeptide.

Fragments or analogs of antibodies can be readily prepared by those of ordinary skill in the art using techniques well-known in the art, and such fragments or analogs are contemplated for use with the formulations of the present invention.

### Formulations

The present invention relates to protein formulations, and in particular, to stable, solid protein formulations as defined by the claims.

Given that certain aspects of the invention are directed to protein formulations that can be stored and used in a variety of environmental conditions (e.g., wide temperature ranges), the invention provides such formulations that maintain protein stability in a variety of these environmental conditions. In addition to the specific formulation constituents set forth herein, the formulations of the invention may further comprise one or more other pharmaceutically acceptable carriers, excipients or stabilizers such as those described in Remington's Pharmaceutical Sciences 16th edition, Osol, A. Ed. (1980), provided that they do not adversely affect the desired characteristics of the formulation.

The formulation comprises an amino acid as a stabilizer, which is histidine. Concentrations of amino acid stabilizers in the formulations of the invention can range from 0.1 mM to 100 mM prior to being solidified, depending on desired properties. In one embodiment, the formulation does not comprise an additional stabilizing protein (e.g., albumin).

The concentration of histidine is 10 mM.

The concentration of histidine is 10 mM.

In further embodiments, the formulations may comprise a buffer that is not an amino acid. In a specific embodiment, the buffer that is not an amino acid is sodium succinate. Concentrations of buffers that are not amino acids in the formulations of the invention can range from 0.1 mM to 100 mM mM prior to being solidified,depending on desired properties.

In one embodiment, the concentration of sodium succinate is 0.1mM to 100 mM. In another embodiment, the concentration of sodium succinate is 0.1 mM to 50 mM. In another embodiment, the concentration of sodium succinate is 0.1 mM to 40 mM. In another embodiment, the concentration of sodium succinate is 0.1 mM to 30 mM. In another embodiment, the concentration of sodium succinate is 0.1 mM to 20 mM. In another embodiment, the concentration of sodium succinate is 0.1 mM to 15 mM. In another embodiment, the concentration of sodium succinate is 0.1 mM to 10 mM. In another embodiment, the concentration of sodium succinate is 0.1 mM to 5 mM. In another embodiment, the concentration of sodium succinate is 0.1 mM to 1 mM. In another embodiment, the concentration of sodium succinate is 1 mM to 100 mM. In another embodiment, the concentration of sodium succinate is 1 mM to 50 mM. In another embodiment, the concentration of sodium succinate is 1 mM to 40 mM. In another embodiment, the concentration of sodium succinate is 1 mM to 30 mM. In another embodiment, the concentration of sodium succinate is 1 mM to 20 mM. In another embodiment, the concentration of sodium succinate is 1 mM to 15 mM. In another embodiment, the concentration of sodium succinate is 1 mM to 10 mM. In another embodiment, the concentration of sodium succinate is 5 mM to 50 mM. In another embodiment, the concentration of sodium succinate is 5 mM to 25 mM. In another embodiment, the concentration of sodium succinate is 5 mM to 15 mM. In another embodiment, the concentration of sodium succinate is 0.1 mM. In another embodiment, the concentration of sodium succinate is 0.5 mM. In another embodiment, the concentration of sodium succinate is 1 mM. In another embodiment, the concentration of sodium succinate is 2 mM. In another embodiment, the concentration of sodium succinate is 3 mM. In another embodiment, the concentration of sodium succinate is 4 mM. In another embodiment, the concentration of sodium succinate is 5 mM. In another embodiment, the concentration of sodium succinate is 6 mM. In another embodiment, the concentration of sodium succinate is 7 mM. In another embodiment, the concentration of sodium succinate is 8 mM. In another embodiment, the concentration of sodium succinate is 9 mM. In another embodiment, the concentration of sodium succinate is 10 mM. In another embodiment, the concentration of sodium succinate is 11 mM. In another embodiment, the concentration of sodium succinate is 12 mM. In another embodiment, the concentration of sodium succinate is 13 mM. In another embodiment, the concentration of sodium succinate is 14 mM. In another embodiment, the concentration of sodium succinate is 15 mM.

In one embodiment, the concentration of sodium succinate is about 0.1mM to about 100 mM. In another embodiment, the concentration of sodium succinate is about 0.1 mM to about 50 mM. In another embodiment, the concentration of sodium succinate is about 0.1 mM to about 40 mM. In another embodiment, the concentration of sodium succinate is about 0.1 mM to about 30 mM. In another embodiment, the concentration of sodium succinate is about 0.1 mM to about 20 mM. In another embodiment, the concentration of sodium succinate is about 0.1 mM to about 15 mM. In another embodiment, the concentration of sodium succinate is about 0.1 mM to about 10 mM. In another embodiment, the concentration of sodium succinate is about 0.1 mM to about 5 mM. In another embodiment, the concentration of sodium succinate is about 0.1 mM to about 1 mM. In another embodiment, the concentration of sodium succinate is about 1 mM to about 100 mM. In another embodiment, the concentration of sodium succinate is about 1 mM to about 50 mM. In another embodiment, the concentration of sodium succinate is about 1 mM to about 40 mM. In another embodiment, the concentration of sodium succinate is about 1 mM to about 30 mM. In another embodiment, the concentration of sodium succinate is about 1 mM to about 20 mM. In another embodiment, the concentration of sodium succinate is about 1 mM to about 15 mM. In another embodiment, the concentration of sodium succinate is about 1 mM to about 10 mM. In another embodiment, the concentration of sodium succinate is about 5 mM to about 50 mM. In another embodiment, the concentration of sodium succinate is about 5 mM to about 25 mM. In another embodiment, the concentration of sodium succinate is about 5 mM to about 15 mM. In another embodiment, the concentration of sodium succinate is about 0.1 mM. In another embodiment, the concentration of sodium succinate is about 0.5 mM. In another embodiment, the concentration of sodium succinate is about 1 mM. In another embodiment, the concentration of sodium succinate is about 2 mM. In another embodiment, the concentration of sodium succinate is about 3 mM. In another embodiment, the concentration of sodium succinate is about 4 mM. In another embodiment, the concentration of sodium succinate is about 5 mM. In another embodiment, the concentration of sodium succinate is about 6 mM. In another embodiment, the concentration of sodium succinate is about 7 mM. In another embodiment, the concentration of sodium succinate is about 8 mM. In another embodiment, the concentration of sodium succinate is about 9 mM. In another embodiment, the concentration of sodium succinate is about 10 mM. In another embodiment, the concentration of sodium succinate is about 11 mM. In another embodiment, the concentration of sodium succinate is about 12 mM. In another embodiment, the concentration of sodium succinate is about 13 mM. In another embodiment, the concentration of sodium succinate is about 14 mM. In another embodiment, the concentration of sodium succinate is about 15 mM.

In further embodiments, the buffer is sodium glutamate. Concentrations of sodium glutamate can range from 0.1 mM to 100 mM mM prior to being solidified,depending on desired properties.

In one embodiment, the concentration of sodium glutamate is 0.1mM to 100 mM. In another embodiment, the concentration of sodium glutamate is 0.1 mM to 50 mM. In another embodiment, the concentration of sodium glutamate is 0.1 mM to 40 mM. In another embodiment, the concentration of sodium glutamate is 0.1 mM to 30 mM. In another embodiment, the concentration of sodium glutamate is 0.1 mM to 20 mM. In another embodiment, the concentration of sodium glutamate is 0.1 mM to 15 mM. In another embodiment, the concentration of sodium glutamate is 0.1 mM to 10 mM. In another embodiment, the concentration of sodium glutamate is 0.1 mM to 5 mM. In another embodiment, the concentration of sodium glutamate is 0.1 mM to 1 mM. In another embodiment, the concentration of sodium glutamate is 1 mM to 100 mM. In another embodiment, the concentration of sodium glutamate is 1 mM to 50 mM. In another embodiment, the concentration of sodium glutamate is 1 mM to 40 mM. In another embodiment, the concentration of sodium glutamate is 1 mM to 30 mM. In another embodiment, the concentration of sodium glutamate is 1 mM to 20 mM. In another embodiment, the concentration of sodium glutamate is 1 mM to 15 mM. In another embodiment, the concentration of sodium glutamate is 1 mM to 10 mM. In another embodiment, the concentration of sodium glutamate is 5 mM to 50 mM. In another embodiment, the concentration of sodium glutamate is 5 mM to 25 mM. In another embodiment, the concentration of sodium glutamate is 5 mM to 15 mM. In another embodiment, the concentration of sodium glutamate is 0.1 mM. In another embodiment, the concentration of sodium glutamate is 0.5 mM. In another embodiment, the concentration of sodium glutamate is 1 mM. In another embodiment, the concentration of sodium glutamate is 2 mM. In another embodiment, the concentration of sodium glutamate is 3 mM. In another embodiment, the concentration of sodium glutamate is 4 mM. In another embodiment, the concentration of sodium glutamate is 5 mM. In another embodiment, the concentration of sodium glutamate is 6 mM. In another embodiment, the concentration of sodium glutamate is 7 mM. In another embodiment, the concentration of sodium glutamate is 8 mM. In another embodiment, the concentration of sodium glutamate is 9 mM. In another embodiment, the concentration of sodium glutamate is 10 mM. In another embodiment, the concentration of sodium glutamate is 11 mM. In another embodiment, the concentration of sodium glutamate is 12 mM. In another embodiment, the concentration of sodium glutamate is 13 mM. In another embodiment, the concentration of sodium glutamate is 14 mM. In another embodiment, the concentration of sodium glutamate is 15 mM.

In one embodiment, the concentration of sodium glutamate is about 0.1mM to about 100 mM. In another embodiment, the concentration of sodium glutamate is about 0.1 mM to about 50 mM. In another embodiment, the concentration of sodium glutamate is about 0.1 mM to about 40 mM. In another embodiment, the concentration of sodium glutamate is about 0.1 mM to about 30 mM. In another embodiment, the concentration of sodium glutamate is about 0.1 mM to about 20 mM. In another embodiment, the concentration of sodium glutamate is about 0.1 mM to about 15 mM. In another embodiment, the concentration of sodium glutamate is about 0.1 mM to about 10 mM. In another embodiment, the concentration of sodium glutamate is about 0.1 mM to about 5 mM. In another embodiment, the concentration of sodium glutamate is about 0.1 mM to about 1 mM. In another embodiment, the concentration of sodium glutamate is about 1 mM to about 100 mM. In another embodiment, the concentration of sodium glutamate is about 1 mM to about 50 mM. In another embodiment, the concentration of sodium glutamate is about 1 mM to about 40 mM. In another embodiment, the concentration of sodium glutamate is about 1 mM to about 30 mM. In another embodiment, the concentration of sodium glutamate is about 1 mM to about 20 mM. In another embodiment, the concentration of sodium glutamate is about 1 mM to about 15 mM. In another embodiment, the concentration of sodium glutamate is about 1 mM to about 10 mM. In another embodiment, the concentration of sodium glutamate is about 5 mM to about 50 mM. In another embodiment, the concentration of sodium glutamate is about 5 mM to about 25 mM. In another embodiment, the concentration of sodium glutamate is about 5 mM to about 15 mM. In another embodiment, the concentration of sodium glutamate is about 0.1 mM. In another embodiment, the concentration of sodium glutamate is about 0.5 mM. In another embodiment, the concentration of sodium glutamate is about 1 mM. In another embodiment, the concentration of sodium glutamate is about 2 mM. In another embodiment, the concentration of sodium glutamate is about 3 mM. In another embodiment, the concentration of sodium glutamate is about 4 mM. In another embodiment, the concentration of sodium glutamate is about 5 mM. In another embodiment, the concentration of sodium glutamate is about 6 mM. In another embodiment, the concentration of sodium glutamate is about 7 mM. In another embodiment, the concentration of sodium glutamate is about 8 mM. In another embodiment, the concentration of sodium glutamate is about 9 mM. In another embodiment, the concentration of sodium glutamate is about 10 mM. In another embodiment, the concentration of sodium glutamate is about 11 mM. In another embodiment, the concentration of sodium glutamate is about 12 mM. In another embodiment, the concentration of sodium glutamate is about 13 mM. In another embodiment, the concentration of sodium glutamate is about 14 mM. In another embodiment, the concentration of sodium glutamate is about 15 mM.

In further embodiments, the formulations may comprise an inorganic salt or an organic salt. In certain embodiments, these salts function as buffers in the protein formulation. Nonlimiting examples of an inorganic salt include sodium chloride, potassium chloride, calcium chloride, sodium phosphate, potassium phosphate, and sodium hydrogen carbonate. Nonlimiting examples of an organic salt include sodium citrate, potassium citrate and sodium acetate. In a specific embodiment, the inorganic salt is sodium phosphate. Concentrations of inorganic or organic salts in the formulations of the invention can range from 0.1 mM to 100 mM mM prior to being solidified,depending on desired properties.

In one embodiment, the concentration of sodium phosphate is 0.1mM to 100 mM. In another embodiment, the concentration of sodium phosphate is 0.1 mM to 50 mM. In another embodiment, the concentration of sodium phosphate is 0.1 mM to 40 mM. In another embodiment, the concentration of sodium phosphate is 0.1 mM to 30 mM. In another embodiment, the concentration of sodium phosphate is 0.1 mM to 20 mM. In another embodiment, the concentration of sodium phosphate is 0.1 mM to 15 mM. In another embodiment, the concentration of sodium phosphate is 0.1 mM to 10 mM. In another embodiment, the concentration of sodium phosphate is 0.1 mM to 5 mM. In another embodiment, the concentration of sodium phosphate is 0.1 mM to 1 mM. In another embodiment, the concentration of sodium phosphate is 1 mM to 100 mM. In another embodiment, the concentration of sodium phosphate is 1 mM to 50 mM. In another embodiment, the concentration of sodium phosphate is 1 mM to 40 mM. In another embodiment, the concentration of sodium phosphate is 1 mM to 30 mM. In another embodiment, the concentration of sodium phosphate is 1 mM to 20 mM. In another embodiment, the concentration of sodium phosphate is 1 mM to 15 mM. In another embodiment, the concentration of sodium phosphate is 1 mM to 10 mM. In another embodiment, the concentration of sodium phosphate is 5 mM to 50 mM. In another embodiment, the concentration of sodium phosphate is 5 mM to 25 mM. In another embodiment, the concentration of sodium phosphate is 5 mM to 15 mM. In another embodiment, the concentration of sodium phosphate is 0.1 mM. In another embodiment, the concentration of sodium phosphate is 0.5 mM. In another embodiment, the concentration of sodium phosphate is 1 mM. In another embodiment, the concentration of sodium phosphate is 2 mM. In another embodiment, the concentration of sodium phosphate is 3 mM. In another embodiment, the concentration of sodium phosphate is 4 mM. In another embodiment, the concentration of sodium phosphate is 5 mM. In another embodiment, the concentration of sodium phosphate is 6 mM. In another embodiment, the concentration of sodium phosphate is 7 mM. In another embodiment, the concentration of sodium phosphate is 8 mM. In another embodiment, the concentration of sodium phosphate is 9 mM. In another embodiment, the concentration of sodium phosphate is 10 mM. In another embodiment, the concentration of sodium phosphate is 11 mM. In another embodiment, the concentration of sodium phosphate is 12 mM. In another embodiment, the concentration of sodium phosphate is 13 mM. In another embodiment, the concentration of sodium phosphate is 14 mM. In another embodiment, the concentration of sodium phosphate is 15 mM.

In one embodiment, the concentration of sodium phosphate is about 0.1mM to about 100 mM. In another embodiment, the concentration of sodium phosphate is about 0.1 mM to about 50 mM. In another embodiment, the concentration of sodium phosphate is about 0.1 mM to about 40 mM. In another embodiment, the concentration of sodium phosphate is about 0.1 mM to about 30 mM. In another embodiment, the concentration of sodium phosphate is about 0.1 mM to about 20 mM. In another embodiment, the concentration of sodium phosphate is about 0.1 mM to about 15 mM. In another embodiment, the concentration of sodium phosphate is about 0.1 mM to about 10 mM. In another embodiment, the concentration of sodium phosphate is about 0.1 mM to about 5 mM. In another embodiment, the concentration of sodium phosphate is about 0.1 mM to about 1 mM. In another embodiment, the concentration of sodium phosphate is about 1 mM to about 100 mM. In another embodiment, the concentration of sodium phosphate is about 1 mM to about 50 mM. In another embodiment, the concentration of sodium phosphate is about 1 mM to about 40 mM. In another embodiment, the concentration of sodium phosphate is about 1 mM to about 30 mM. In another embodiment, the concentration of sodium phosphate is about 1 mM to about 20 mM. In another embodiment, the concentration of sodium phosphate is about 1 mM to about 15 mM. In another embodiment, the concentration of sodium phosphate is about 1 mM to about 10 mM. In another embodiment, the concentration of sodium phosphate is about 5 mM to about 50 mM. In another embodiment, the concentration of sodium phosphate is about 5 mM to about 25 mM. In another embodiment, the concentration of sodium phosphate is about 5 mM to about 15 mM. In another embodiment, the concentration of sodium phosphate is about 0.1 mM. In another embodiment, the concentration of sodium phosphate is about 0.5 mM. In another embodiment, the concentration of sodium phosphate is about 1 mM. In another embodiment, the concentration of sodium phosphate is about 2 mM. In another embodiment, the concentration of sodium phosphate is about 3 mM. In another embodiment, the concentration of sodium phosphate is about 4 mM. In another embodiment, the concentration of sodium phosphate is about 5 mM. In another embodiment, the concentration of sodium phosphate is about 6 mM. In another embodiment, the concentration of sodium phosphate is about 7 mM. In another embodiment, the concentration of sodium phosphate is about 8 mM. In another embodiment, the concentration of sodium phosphate is about 9 mM. In another embodiment, the concentration of sodium phosphate is about 10 mM. In another embodiment, the concentration of sodium phosphate is about 11 mM. In another embodiment, the concentration of sodium phosphate is about 12 mM. In another embodiment, the concentration of sodium phosphate is about 13 mM. In another embodiment, the concentration of sodium phosphate is about 14 mM. In another embodiment, the concentration of sodium phosphate is about 15 mM.

The protein formulation comprises a sugar alcohol, which is mannitol. The concentration of mannitol is about 2%.

The formulation comprises a sugar which is sucrose. The concentration of sucrose is about 0.5%.

The formulation comprises a surfactant, which is polysorbate-80. The concentration of polysorbate 80 is about 0.005%.

In one embodiment, the concentration of polysorbate 20 is between about 0.0001% and about 0.1%. In another embodiment, the concentration of polysorbate 20 is between about 0.001% and about 0.5%. In another embodiment, the concentration of polysorbate 20 is between about 0.001% and about 0.1%. In another embodiment, the concentration of polysorbate 20 is between about 0.005% and about 0.1%. In another embodiment, the concentration of polysorbate 20 is between about 0.005% and about 0.05%. In another embodiment, the concentration of polysorbate 20 is between about 0.01% and about 0.1%. In another embodiment, the concentration of polysorbate 20 is between about 0.5% and about 0.1%. In another embodiment, the concentration of polysorbate 20 is about 0.009%. In another embodiment, the concentration of polysorbate 20 is about 0.008%. In another embodiment, the concentration of polysorbate 20 is about 0.007%. In another embodiment, the concentration of polysorbate 20 is about 0.006%. In another embodiment, the concentration of polysorbate 20 is about 0.005%. In another embodiment, the concentration of polysorbate 20 is about 0.004%. In another embodiment, the concentration of polysorbate 20 is about 0.003%. In another embodiment, the concentration of polysorbate 20 is about 0.002%. In another embodiment, the concentration of polysorbate 20 is about 0.001%. In another embodiment, the concentration of polysorbate 20 is about 0.01%. In another embodiment, the concentration of polysorbate 20 is about 0.02%. In another embodiment, the concentration of polysorbate 20 is about 0.03%. In another embodiment, the concentration of polysorbate 20 is about 0.04%. In another embodiment, the concentration of polysorbate 20 is about 0.05%. In another embodiment, the concentration of polysorbate 20 is about 0.06%. In another embodiment, the concentration of polysorbate 20 is about 0.07%. In another embodiment, the concentration of polysorbate 20 is about 0.08%. In another embodiment, the concentration of polysorbate 20 is about 0.09%. In another embodiment, the concentration of polysorbate 20 is about 0.1%. In another embodiment, the concentration of polysorbate 20 is about 0.5%.

In one embodiment, the concentration of polysorbate 20 is between 0.0001% and 0.01%. In another embodiment, the concentration of polysorbate 20 is between 0.001% and 0.5%. In another embodiment, the concentration of polysorbate 20 is between 0.001% and 0.1%. In another embodiment, the concentration of polysorbate 20 is between 0.005% and 0.1%. In another embodiment, the concentration of polysorbate 20 is between 0.005% and 0.05%. In another embodiment, the concentration of polysorbate 20 is between 0.01% and .1%. In another embodiment, the concentration of polysorbate 20 is between 0.5% and 0.1%. In another embodiment, the concentration of polysorbate 20 is 0.009%. In another embodiment, the concentration of polysorbate 20 is 0.008%. In another embodiment, the concentration of polysorbate 20 is 0.007%. In another embodiment, the concentration of polysorbate 20 is 0.006%. In another embodiment, the concentration of polysorbate 20 is 0.005%. In another embodiment, the concentration of polysorbate 20 is 0.004%. In another embodiment, the concentration of polysorbate 20 is 0.003%. In another embodiment, the concentration of polysorbate 20 is 0.002%. In another embodiment, the concentration of polysorbate 20 is 0.001%. In another embodiment, the concentration of polysorbate 20 is 0.01%. In another embodiment, the concentration of polysorbate 20 is 0.02%. In another embodiment, the concentration of polysorbate 20 is 0.03%. In another embodiment, the concentration of polysorbate 20 is 0.04%. In another embodiment, the concentration of polysorbate 20 is 0.05%. In another embodiment, the concentration of polysorbate 20 is 0.06%. In another embodiment, the concentration of polysorbate 20 is 0.07%. In another embodiment, the concentration of polysorbate 20 is 0.08%. In another embodiment, the concentration of polysorbate 20 is 0.09%. In another embodiment, the concentration of polysorbate 20 is 0.1%. In another embodiment, the concentration of polysorbate 20 is 0.5%.

In one embodiment, the pH is between about 5.0 and about 8.0 prior to being solidified. In another embodiment, the pH is between about 4.0 and about 10.0. In another embodiment, the pH is between about 5.0 and about 7.0. In another embodiment, the pH is between about 5.0 and about 9.0. In another embodiment, the pH is between about 6.0 and about 9.0. In another embodiment, the pH is about 5.0. In another embodiment, the pH is about 5.5. In another embodiment, the pH is about 6.0. In another embodiment, the pH is about 6.5. In another embodiment, the pH is about 7.0. In another embodiment, the pH is about 7.5. In another embodiment, the pH is about 8.0. In another embodiment, the pH is about 8.5. In another embodiment, the pH is about 9.0. In another embodiment, the pH is about 9.5. In another embodiment, the pH is about 10.0.

In one embodiment, the pH is between 6.0 and 8.0 prior to being solidified. In another embodiment, the pH is between 4.0 and 10.0. In another embodiment, the pH is between 5.0 and 7.0. In another embodiment, the pH is between 5.0 and 9.0. In another embodiment, the pH is between 6.0 and 9.0. In another embodiment, the pH is 5.0. In another embodiment, the pH is 5.1. In another embodiment, the pH is 5.2. In another embodiment, the pH is 5.3. In another embodiment, the pH is 5.4. In another embodiment, the pH is 5.5. In another embodiment, the pH is 5.6. In another embodiment, the pH is 5.7. In another embodiment, the pH is 5.8. In another embodiment, the pH is 5.9. In another embodiment, the pH is 6.0. In another embodiment, the pH is 6.1. In another embodiment, the pH is 6.2. In another embodiment, the pH is 6.3. In another embodiment, the pH is 6.4. In another embodiment, the pH is 6.5. In another embodiment, the pH is 6.6. In another embodiment, the pH is 6.7. In another embodiment, the pH is 6.8. In another embodiment, the pH is 6.9. In another embodiment, the pH is 7.0. In another embodiment, the pH is 7.5. In another embodiment, the pH is 8.0. In another embodiment, the pH is 8.5. In another embodiment, the pH is 9.0. In another embodiment, the pH is 9.5. In another embodiment, the pH is 10.0.

In one embodiment, the concentration of protein is between about 0.01 mg/ml and about 10 mg/ml. In one embodiment, the concentration of protein is between about 0.01 mg/ml and about 1 mg/ml. In one embodiment, the concentration of protein is between about 0.1 mg/ml and about 100 mg/ml mM prior to being solidified. In another embodiment, the concentration of protein is between about 1 mg/ml and about 100 mg/ml. In another embodiment, the concentration of protein is between about 1 mg/ml and about 50 mg/ml. In another embodiment, the concentration of protein is between about 1 mg/ml and about 25 mg/ml. In another embodiment, the concentration of protein is between about 1 mg/ml and about 10 mg/ml. In another embodiment, the concentration of protein is between about 5 mg/ml and about 50 mg/ml. In another embodiment, the concentration of protein is between about 5 mg/ml and about 25 mg/ml. In another embodiment, the concentration of protein is between about 10 mg/ml and about 100 mg/ml. In another embodiment, the concentration of protein is between about 10 mg/ml and about 50 mg/ml. In another embodiment, the concentration of protein is between about 10 mg/ml and about 25 mg/ml. In another embodiment, the concentration of protein is about 0.01 mg/ml. In another embodiment, the concentration of protein is about 0.05 mg/ml. In another embodiment, the concentration of protein is about 0.1 mg/ml. In another embodiment, the concentration of protein is about 0.2 mg/ml. In another embodiment, the concentration of protein is about 0.3 mg/ml. In another embodiment, the concentration of protein is about 0.4 mg/ml. In another embodiment, the concentration of protein is about 0.5 mg/ml. In another embodiment, the concentration of protein is about 1 mg/ml. In another embodiment, the concentration of protein is about 2 mg/ml. In another embodiment, the concentration of protein is about 3 mg/ml. In another embodiment, the concentration of protein is about 4 mg/ml. In another embodiment, the concentration of protein is about 5 mg/ml. In another embodiment, the concentration of protein is about 6 mg/ml. In another embodiment, the concentration of protein is about 7 mg/ml. In another embodiment, the concentration of protein is about 8 mg/ml. In another embodiment, the concentration of protein is about 9 mg/ml. In another embodiment, the concentration of protein is about 10 mg/ml. In another embodiment, the concentration of protein is about 20 mg/ml. In another embodiment, the concentration of protein is about 30 mg/ml. In another embodiment, the concentration of protein is about 40 mg/ml. In another embodiment, the concentration of protein is about 50 mg/ml. In another embodiment, the concentration of protein is about 60 mg/ml. In another embodiment, the concentration of protein is about 70 mg/ml. In another embodiment, the concentration of protein is about 80 mg/ml. In another embodiment, the concentration of protein is about 90 mg/ml. In another embodiment, the concentration of protein is about 100 mg/ml.

In one embodiment, the concentration of protein is between 0.01 mg/ml and 10 mg/ml. In one embodiment, the concentration of protein is between 0.01 mg/ml and 1 mg/ml. In one embodiment, the concentration of protein is between 0.1 mg/ml and 100 mg/ml mM prior to being solidified. In another embodiment, the concentration of protein is between 1 mg/ml and 100 mg/ml. In another embodiment, the concentration of protein is between 1 mg/ml and 50 mg/ml. In another embodiment, the concentration of protein is between 1 mg/ml and 25 mg/ml. In another embodiment, the concentration of protein is between 1 mg/ml and 10 mg/ml. In another embodiment, the concentration of protein is between 5 mg/ml and 50 mg/ml. In another embodiment, the concentration of protein is between 5 mg/ml and 25 mg/ml. In another embodiment, the concentration of protein is between 10 mg/ml and 100 mg/ml. In another embodiment, the concentration of protein is between 10 mg/ml and 50 mg/ml. In another embodiment, the concentration of protein is between 10 mg/ml and 25 mg/ml. In another embodiment, the concentration of protein is 0.01 mg/ml. In another embodiment, the concentration of protein is 0.05 mg/ml. In another embodiment, the concentration of protein is 0.1 mg/ml. In another embodiment, the concentration of protein is 0.2 mg/ml. In another embodiment, the concentration of protein is 0.3 mg/ml. In another embodiment, the concentration of protein is 0.4 mg/ml. In another embodiment, the concentration of protein is 0.5 mg/ml. In another embodiment, the concentration of protein is 1 mg/ml. In another embodiment, the concentration of protein is 2 mg/ml. In another embodiment, the concentration of protein is 3 mg/ml. In another embodiment, the concentration of protein is 4 mg/ml. In another embodiment, the concentration of protein is 5 mg/ml. In another embodiment, the concentration of protein is 6 mg/ml. In another embodiment, the concentration of protein is 7 mg/ml. In another embodiment, the concentration of protein is 8 mg/ml. In another embodiment, the concentration of protein is 9 mg/ml. In another embodiment, the concentration of protein is 10 mg/ml. In another embodiment, the concentration of protein is 20 mg/ml. In another embodiment, the concentration of protein is 30 mg/ml. In another embodiment, the concentration of protein is 40 mg/ml. In another embodiment, the concentration of protein is 50 mg/ml. In another embodiment, the concentration of protein is 60 mg/ml. In another embodiment, the concentration of protein is 70 mg/ml. In another embodiment, the concentration of protein is 80 mg/ml. In another embodiment, the concentration of protein is 90 mg/ml. In another embodiment, the concentration of protein is 100 mg/ml.

Solid protein formulations encompass, but are not limited to, protein formulations that start off in a liquid state and subsequently have the liquid removed (i.e., progressing from a hydrated protein solution to a dehydrated protein solution). Nonlimiting examples of methods to achieve this include lyophilization (also called freeze-drying or cryodesication) or spray drying. In lyophilization, the material is rapidly frozen and dehydrated while under vacuum conditions, resulting in sublimation of the liquid from solid phase to a gaseous phase. Spray drying is a method of producing a dry powder from a liquid or slurry by rapidly drying with a hot gas. The processes of lyophilization and spray drying are well known in the art and can readily be optimized to produce the desired outcome. See also Lyophilization of Biopharmaceuticals, Biotechnology: Pharmaceutical Aspects, Henry R. Costantino (Editor), Michael J. Pikal (Editor); Lyophilization: Introduction and Basic Principles, Thomas A. Jennings, CRC Press, 1999; and Freeze-Drying/Lyophilization Of Pharmaceutical & Biological Products, Second Edition: Revised And Expanded (Drugs and the Pharmaceutical Sciences), Louis Rey (Editor), Joan C. May (Editor), CRC Press 2004. In one embodiment, the solid protein formulation is lyophilized. In another embodiment, the solid protein formulation is spray dried. Throughout this disclosure, there may be reference to "prior to being solidified", which means the protein formulation in liquid state prior to having the liquid removed as described above.

One aspect of the invention is to provide protein formulations (both solid and liquid) that are stable, and maintain their stability through a variety of different temperatures and for extended periods of time. This property is useful for variety of different reasons, including, but not limited to, ease of storage in a wide variety of clinical settings.

In one embodiment, the solid protein formulation is stable for at least one month at between 4 degrees C and 45 degrees C. In one embodiment, the solid protein formulation is stable for at least two months at between 4 degrees C and 45 degrees C. In one embodiment, the solid protein formulation is stable for at least three months at between 4 degrees C and 45 degrees C. In one embodiment, the solid protein formulation is stable for at least four months at between 4 degrees C and 45 degrees C. In one embodiment, the solid protein formulation is stable for at least five months at between 4 degrees C and 45 degrees C. In one embodiment, the solid protein formulation is stable for at least six months at between 4 degrees C and 45 degrees C. In one embodiment, the solid protein formulation is stable for at least twelve months at between 4 degrees C and 45 degrees C. In one embodiment, the solid protein formulation is stable for at least eighteen months at between 4 degrees C and 45 degrees C. In one embodiment, the solid protein formulation is stable for at least twenty-four months at between 4 degrees C and 45 degrees C. In one embodiment, the solid protein formulation is stable for at least thirty-six months at between 4 degrees C and 45 degrees C. In one embodiment, the solid protein formulation is stable for at least forty-eight months at between 4 degrees C and 45 degrees C. In one embodiment, the solid protein formulation is stable for at least sixty months at between 4 degrees C and 45 degrees C.

In one embodiment, the solid protein formulation is stable for at least about one month at between about 4 degrees C and about 45 degrees C. In one embodiment, the solid protein formulation is stable for at least about two months at between about 4 degrees C and about 45 degrees C. In one embodiment, the solid protein formulation is stable for at least about three months at between about 4 degrees C and about 45 degrees C. In one embodiment, the solid protein formulation is stable for at least about four months at between about 4 degrees C and about 45 degrees C. In one embodiment, the solid protein formulation is stable for at least about five months at between about 4 degrees C and about 45 degrees C. In one embodiment, the solid protein formulation is stable for at least about six months at between about 4 degrees C and about 45 degrees C. In one embodiment, the solid protein formulation is stable for at least about twelve months at between about 4 degrees C and about 45 degrees C. In one embodiment, the solid protein formulation is stable for at least about eighteen months at between about 4 degrees C and about 45 degrees C. In one embodiment, the solid protein formulation is stable for at least about twenty-four months at between about 4 degrees C and about 45 degrees C. In one embodiment, the solid protein formulation is stable for at least about thirty-six months at between about 4 degrees C and about 45 degrees C. In one embodiment, the solid protein formulation is stable for at least about forty-eight months at between about 4 degrees C and about 45 degrees C. In one embodiment, the solid protein formulation is stable for at least about sixty months at between about 4 degrees C and about 45 degrees C.

In one embodiment, the solid protein formulation is stable for at least about one month at about 4 degrees C, about 29 degrees C, about 37 degrees C, and/or about 45 degrees C. In a further embodiment, the solid protein formulation is stable for at least about two months at about 4 degrees C, about 29 degrees C, about 37 degrees C, and/or about 45 degrees C. In a further embodiment, the solid protein formulation is stable for at least about three months at about 4 degrees C, about 29 degrees C, about 37 degrees C, and/or about 45 degrees C. In a further embodiment, the solid protein formulation is stable for at least about four months at about 4 degrees C, about 29 degrees C, about 37 degrees C, and/or about 45 degrees C. In a further embodiment, the solid protein formulation is stable for at least about five months at about 4 degrees C, about 29 degrees C, about 37 degrees C, and/or about 45 degrees C. In a further embodiment, the solid protein formulation is stable for at least about six months at about 4 degrees C, about 29 degrees C, about 37 degrees C, and/or about 45 degrees C. In a further embodiment, the solid protein formulation is stable for at least about twelve months at about 4 degrees C, about 29 degrees C, about 37 degrees C, and/or about 45 degrees C. In a further embodiment, the solid protein formulation is stable for at least about eighteen months at about 4 degrees C, about 29 degrees C, about 37 degrees C, and/or about 45 degrees C. In a further embodiment, the solid protein formulation is stable for at least about twenty-four months at about 4 degrees C, about 29 degrees C, about 37 degrees C, and/or about 45 degrees C. In a further embodiment, the solid protein formulation is stable for at least about thirty-six months at about 4 degrees C, about 29 degrees C, about 37 degrees C, and/or about 45 degrees C. In a further embodiment, the solid protein formulation is stable for at least about fourty-eight months at about 4 degrees C, about 29 degrees C, about 37 degrees C, and/or about 45 degrees C. In a further embodiment, the solid protein formulation is stable for at least about sixty months at about 4 degrees C, about 29 degrees C, about 37 degrees C, and/or about 45 degrees C.

In yet a further embodiment, the solid protein formulation is reconstituted with a liquid. In one embodiment, the solid protein formulation is reconstituted with water. In another embodiment, the solid protein formulation is reconstituted with a reconstitution solution comprising sorbitol and sodium chloride. In certain embodiments, the concentration of the sorbitol is 4%, the concentration of sodium chloride is 0.7% and the pH is 7. In further embodiments, the solid protein formulation is reconstituted with a reconstitution solution that further comprises benzyl alcohol. In a specific emboment, the concentration of benzyl alcohol is 1%. In further embodiments, the liquid used to reconstitute the solid protein formulation is sterile.

In a specific embodiment, the invention provides a solid protein formulation consisting of, consisting essentially of, or comprising: Aranesp (0.5 to 5 mg/ml), 10 mM histidine, 2% mannitol, 0.5% sucrose, 0.005% polysorbate-80, at pH 7, prior to being solidified.

In a specific embodiment, the invention provides a solid protein formulation consisting of, consisting essentially of, or comprising: Aranesp (2 mg/ml), 10 mM histidine, 2% mannitol, 0.5% sucrose, 0.005% polysorbate-80, at pH 7, prior to being solidified.

In a specific embodiment, the invention provides a solid protein formulation consisting of, consisting essentially of, or comprising: Aranesp (0.5 to 5 mg/ml), 10 mM histidine, 2% mannitol, 0.5% sucrose, 0.005% polysorbate-80, at pH 7, prior to being solidified.

Also disclosed, but not according to the invention is a solid protein formulation consisting of, consisting essentially of, or comprising: Aranesp (0.5 to 5 mg/ml), 10 mM sodium phosphate, 4.5% mannitol, 0.5% sucrose, 0.005% polysorbate-80, at pH 6, prior to being solidified.

Also disclosed, but not according to the invention is a solid protein formulation consisting of, consisting essentially of, or comprising: Aranesp (0.5 to 5 mg/ml), 10 mM sodium phosphate, 2% mannitol, 0.5% sucrose, 0.005% polysorbate-80, at pH 7, prior to being solidified.

Also disclosed, but not according to the invention is a solid protein formulation consisting of, consisting essentially of, or comprising: Aranesp (0.5 to 5 mg/ml), 10 mM sodium succinate, 2% mannitol, 0.5% sucrose, 0.005% polysorbate-80, at pH 7, prior to being solidified.

Also disclosed, but not according to the invention is a solid protein formulation consisting of, consisting essentially of, or comprising: Aranesp (0.5 to 5 mg/ml), 10 mM sodium succinate, 4.5% mannitol, 0.5% sucrose, 0.005% polysorbate-80, at pH 6, prior to being solidified.

Also disclosed, but not according to the invention is a solid protein formulation consisting of, consisting essentially of, or comprising: Aranesp (0.5 to 5 mg/ml), 20 mM sodium phosphate, 140 mM NaCl, 0.005% polysorbate-80, at pH 6.2, prior to being solidified.

Also disclosed, but not according to the invention is a solid protein formulation consisting of, consisting essentially of, or comprising: Aranesp (0.5 to 5 mg/ml), 10 mM histidine, 2% mannitol, 0.5% sucrose at pH 7, prior to being solidified.

Also disclosed, but not according to the invention is a solid protein formulation consisting of, consisting essentially of, or comprising: Aranesp (2 mg/ml), 10 mM histidine, 2% mannitol, 0.5% sucrose at pH 7, prior to being solidified.

Also disclosed, but not according to the invention is a solid protein formulation consisting of, consisting essentially of, or comprising: Aranesp (0.5 to 5 mg/ml), 10 mM histidine, 2% mannitol, 0.5% sucrose at pH 7, prior to being solidified.

Also disclosed, but not according to the invention is a solid protein formulation consisting of, consisting essentially of, or comprising: Aranesp (0.5 to 5 mg/ml), 10 mM sodium phosphate, 4.5% mannitol, 0.5% sucrose at pH 6, prior to being solidified.

Also disclosed but not according to the invention is a solid protein formulation consisting of, consisting essentially of, or comprising: Aranesp (0.5 to 5 mg/ml), 10 mM sodium phosphate, 2% mannitol, 0.5% sucrose at pH 7, prior to being solidified.

Also disclosed but not according to the invention is a solid protein formulation consisting of, consisting essentially of, or comprising: Aranesp (0.5 to 5 mg/ml), 10 mM sodium succinate, 2% mannitol, 0.5% sucrose at pH 7, prior to being solidified.

Also disclosed but not according to the invention is a solid protein formulation consisting of, consisting essentially of, or comprising: Aranesp (0.5 to 5 mg/ml), 10 mM sodium succinate, 4.5% mannitol, 0.5% sucrose at pH 6, prior to being solidified.

Also disclosed but not according to the invention is a solid protein formulation consisting of, consisting essentially of, or comprising: Aranesp (0.5 to 5 mg/ml), 20 mM sodium phosphate, 140 mM NaCl at pH 6.2, prior to being solidified.

In another embodiment, the invention provides a method of preparing a stable, solid protein formulation, said method comprising: a) diluting said protein with a lyophilization buffer; and b) lyophilizing said diluted protein, prior to being solidified.

In certain embodiments, the formulations of the invention comprise a glycoprotein. In one embodiment, the protein is erythropoietin. In another embodiment, the protein is an analog of erythropoietin. In a further embodiment, the protein is darbepoetin alfa. In yet a further embodiment, the protein is an antibody. In yet another embodiment, the protein is a monoclonal antibody or a polyclonal antibody.

Erythropoietin is a glycoprotein hormone involved in the maturation of erythroid progenitor cells into erythrocytes. It is essential in regulating levels of red blood cells in circulation. Naturally occurring erythropoietin is produced by the liver during fetal life and by the kidney of adults and circulates in the blood and stimulates the production of red blood cells in bone marrow. Anemia is almost invariably a consequence of renal failure due to decreased production of erythropoietin from the kidney. Recombinant erythropoietin produced by genetic engineering techniques involving the expression of a protein product from a host cell transformed with the gene encoding erythropoietin has been found to be effective when used in the treatment of anemia resulting from chronic renal failure.

The identification, cloning, and expression of genes encoding erythropoietin are described in U.S. Pat. No. 4,703,008. A description of the purification of recombinant erythropoietin from cell medium that supported the growth of mammalian cells containing recombinant erythropoietin plasmids, for example, is included in U.S. Pat. No. 4,667,016. The expression and recovery of biologically active recombinant erythropoietin from mammalian cell hosts containing the erythropoietin gene on recombinant plasmids has made available quantities of erythropoietin suitable for therapeutic applications. The polynucleotide and polypeptide sequences for several species of erythropoietin are known.

Currently, there are several recombinant human erythropoietin drug products on the market (*e.g.*, Epogen^{®}; epoetin alfa). In addition to epoetin alfa, there are other epoetins that have been developed (*e.g*., epoetin beta, delta, omega, zeta). In the context of the present invention, all forms of erythropoietins are meant to be encompassed when the term "erythropoietin" or "recombinant human erythropoietin" are used.

Also encompassed by the invention are certain formulations comprising analogs of human erythropoietin. In certain embodiments, the phrase "analog of human erythropoietin" refers to erythropoietin with one or more changes in the amino acid sequence of human erythropoietin, resulting in an increase in the number of sites for sialic acid attachment. One nonlimiting example is darbepoetin alfa (Aranesp^{®}), a hyperglycosylated analog of human erythropoietin. The added sites for glycosylation in these analogs may result in a greater number of carbohydrate chains, and higher sialic acid content, than human erythropoietin. Erythropoietin analogs comprising amino acid sequences which include the rearrangement of at least one site for glycosylation are also provided. Analogs comprising an addition of one or more amino acids to the carboxy terminal end of erythropoietin wherein the addition provides at least one glycosylation site are also included. Analogs can be generated by site-directed mutagenesis having additions, deletions, or substitutions of amino acid residues that increase or alter sites that are available for glycosylation. Such analogs may have a greater number of carbohydrate chains than human erythropoietin. See additionally, for example, U.S. Patent No. 7,217,689.

In another aspect, the present invention provides formulations comprising antibodies, antibody fragments, antibody derivatives, antibody muteins, and antibody variants, that specifically bind to human erythropoietin.

Antibodies can comprise any constant region known in the art. The light chain constant region can be, for example, a kappa- or lambda-type light chain constant region, e.g., a human kappa- or lambda-type light chain constant region. The heavy chain constant region can be, for example, an alpha-, delta-, epsilon-, gamma-, or mu-type heavy chain constant regions, e.g., a human alpha-, delta-, epsilon-, gamma-, or mu-type heavy chain constant region. In one embodiment, the light or heavy chain constant region is a fragment, derivative, variant, or mutein of a naturally occurring constant region.

In one embodiment, an antibody further comprises the constant light chain kappa or lambda domains or a fragment of these. Sequences of the light chain constant regions and polynucleotides encoding them well known in the art. In another embodiment, an further comprises a heavy chain constant domain, or a fragment thereof, such as the IgG1 or IgG2 heavy chain constant region, such sequences are well known in the art.

Antibodies include those having a desired isotype (for example, IgA, IgG1, IgG2, IgG3, IgG4, IgM, IgE, and IgD) as well as Fab or F(ab')₂ fragments thereof. Moreover, if an IgG4 is desired, it may also be desired to introduce a point mutation in the hinge region as described in Bloom et al., 1997, Protein Science 6:407, (incorporated by reference herein) to alleviate a tendency to form intra-H chain disulfide bonds that can lead to heterogeneity in the IgG4 antibodies.

The term "antibody" refers to an intact antibody, or an antigen binding fragment thereof, as described extensively in the Definitions section. An antibody may comprise a complete antibody molecule (including polyclonal, monoclonal, chimeric, humanized, or human versions having full length heavy and/or light chains), or comprise an antigen binding fragment thereof. Antibody fragments include F(ab')₂, Fab, Fab', Fv, Fc, and Fd fragments, and can be incorporated into single domain antibodies, single-chain antibodies, maxibodies, minibodies, intrabodies, diabodies, triabodies, tetrabodies, v-NAR and bis-scFv (see e.g., Hollinger and Hudson, 2005, Nature Biotechnology, 23, 9, 1126-1136). Also included are antibody polypeptides such as those disclosed in U. S. Patent No. 6,703,199, including fibronectin polypeptide monobodies. Other antibody polypeptides are disclosed in U.S. Patent Publication 2005/0238646, which are single-chain polypeptides.

The invention provides stable protein formulations. The term stable, in certain, nonlimiting embodiments, is in reference to the structure and activity of the protein. In certain embodiments, stable means the protein maintains proper amino acid sequence and conformation structure. Nonlimiting examples of methods to assay this include antibody binding assays or gel electrophoresis.

In certain embodiments, one aspect of stability is the percent oxidation measured over a storage period of time. As a protein oxidized, is can lose activity, among other properties. In one embodiment, the protein in the formulations of the present invention have a percent oxidation of between about 5% and about 10%. In another embodiment, the protein in the formulations of the present invention have a percent oxidation of less than about 10%. In another embodiment, the protein in the formulations of the present invention have a percent oxidation of less than about 5%. In one embodiment, the protein in the formulations of the present invention have a percent oxidation of between 5% and 10%. In another embodiment, the protein in the formulations of the present invention have a percent oxidation of less than 10%. In another embodiment, the protein in the formulations of the present invention have a percent oxidation of less than 5%.

In yet further embodiments, stable means that the protein present in the formulation has and retains a biological effect. Nonlimiting examples include bioassays that measure the effect of the protein on a cell line *in vitro,* or bioassays that measure the effect of the protein on an animal *in vivo.*

The protein formulations of the present invention can be administered to a patient through various routes, and appropriate to the indication and the composition. The protein formulations of the present invention may be administered by any suitable technique, including, but not limited to parenteral administration. If injected, the formulation can be administered, for example, via intra-articular, intravenous, intramuscular, intralesional, intraperitoneal or subcutaneous routes, by bolus injection, or continuous infusion.

Dosages and the frequency of administration may vary according to such factors as the route of administration, the particular protein employed, the nature and severity of the disease to be treated, whether the condition is acute or chronic, and the size and general condition of the subject. Appropriate dosages can be determined by procedures known in the pertinent art, *e.g.,* in clinical trials that may involve dose escalation studies.

Kits for use by medical practitioners and/or subjects are provided including one or more proteins in a formulation of the invention and a label or other instructions for use in treating any of the conditions discussed herein. In one embodiment, the kit includes a sterile preparation of one or more proteins in a lyophilized formulation of the invention, and a separate, sterile reconstituting solution, and these may be in one or more vials.

The invention having been described, the following examples are offered by way of illustration, and not limitation.

### EXAMPLES

### EXAMPLE 1

### 1. Purpose

The purpose of this study was to investigate the feasibility of development of a room temperature stable Aranesp^{®} formulation to have advantage in handling and storage. The current commercial formulation is a liquid formulation that is stored at 2-8C. The lyophilized formulation is not a preferred formulation in the market since it needs to be reconstituted and injection with two step procedure. Since LyoTip^{®}, a device that offers convenient way of delivering lyophilized formulation as liquid pre-filled syringes by combining both the reconstitution and injection process into a seamless single step, lyophilized formulations were investigated for long term shelf stability study for room temperature stable formulation in 3 cc glass vial.

### 2. Summary of Experiments

Aranesp^{®} (2 mg/mL) was formulated in five of lyophilized formulation by dialysis of Aranesp^{®} bulk and the shelf stability was compared to liquid formulation in 20 mM sodium phosphate, 140 mM sodium chloride, pH 6.2. The dialyzed sample (0.25 mL) was filled into a glass 3 cc vial and was lyophilized. The lyophilized samples were stored at 4°C, 29°C, 37°C, and 45 °C for 24 months for shelf stability. At each time point, samples were reconstituted with 1 mL of three reconstitution solutions (Water for P45MSu6 and S45MSu6; 4 % Sorbitol and 0.7% sodium chloride were used for P2MSu7, S2MSu7and H2MSu7) to make final concentration of 500 µg/mL. The reconstituted samples were analyzed for SEC-HPLC (Size Exclusion HPLC), antibody 9G8A (Monitoring relative protein unfolding), pH, % Oxidation, RP-HPLC (impurities and clips), sub-visible particle, Concentration, and Osmolarity.

### 3. Material and Equipment

The following materials were used: Aranesp bulk material (2 mg/mL), Polysorbate 80, glass 3cc vials, Daikyo long stoppers for lyophilization. The following six formulation buffers were prepared:
- P2MSu7: 10 mM Sodium Phosphate, 2 % Mannitol, 0.5 % Sucrose, pH 7 (Lot#29070904-11)
- S2MSu7: 10 mM Sodium Succinate, 2 % Mannitol, 0.5 % Sucrose, pH 7 (Lot# 29070904-21)
- H2MSu7: 10 mM Histidine, 2 % Mannitol, 0.5% Sucrose, pH 7 (Lot# 29070904-19)
- P45MSu6: 10 mM Sodium Phosphate, 4.5 % Mannitol, 0.5% Sucrose, pH 6 (Lot# 29070627-18)
- S45MSu6: 10 mM Sodium Succinate, 4.5 % Mannitol, 0.5% Sucrose, pH 6 (Lot# 29070627-19)
- P62N: 20 mM Sodium Phosphate, 140 mM NaCl, pH 6.2

Additional equipment used included: Slide-A Lyser dialysis cassette (Pierce), Corning filter (0.22 µm), cold room to dialyze samples for 2 days, and a Virtis lyophilizer was used for lyophillization.

### 4. Sample preparation Procedure

### 4.1. Sample preparation

Aranesp^{®} (2 mg/mL) was dialyzed in five formulation buffers in the cold room (2-8 °C) for 2 days using a Slide-A Lyser dialysis cassette. The buffer was checked for pH and osmolarity at the room temperature before dialyzing samples. After dialysis, the concentration of the sample was checked by A280. The samples were prepared with 0.005% polysorbate-80 and without polysorbate-80. The samples with polysorbate-80 were prepared by adding 10% (w/v) polysorbate-80 stock solution to the sample to make 0.005 %. The 10 % polysorbate-80 solution was made with weighing 2.5 g of polysorbate-80 and dissolved with 25 mL of each buffer. The samples were filtered using Corning filter (0.22 µm) in the sterile hood. The filtered sample and placebo (0.25 mL) was filled into 3cc glass vial and was subjected to lyophilized cycle using Virtis lyophilizer (Table 1). The liquid sample in 20 mM sodium phosphate, 140 mM sodium chloride, 0.005% polysorbate-80, pH 6.2 was prepared by adding polysorbate-80 in the bulk to make 0.005%. The liquid control samples, lyophilized samples and placebos were stored at 4 °C, 29 °C, 37 °C, and 45 °C for 24 months for shelf stability. At each time point, samples were reconstituted with 1 mL of three reconstitution solutions (Water for P45MSu6 and S45MSu6; 4% Sorbitol and 0.7 % sodium chloride were used for P2MSu7, S2MSu7and H2MSu7) to make final concentration of 500 µg/mL. When the samples (0.25 mL) with 0.005% polysorbate-80 were reconstituted with 1mL of respective reconstitution buffer, the sample contained 0.00125% of polysorbate-80 since it was diluted with 1 mL reconstitution buffer. The reconstituted samples were analyzed for SEC-HPLC (Size Exclusion HPLC), 9G8A (Monitoring relative protein unfolding), pH, % Oxidation, and Non-reduced RP-HPLC (impurities and clips).

Sample list, time points, and temperature are indicated in Appendix 1.

### 4.2. Lyophilization procedure:

Sample vials were loaded on pre-chilled (4 °C) shelves of a lyophilizer. Samples were subjected for three steps of freezing, primary drying and secondary drying. First the samples were hold for 60 minutes at 4 °C and then cooled down from 4 °C to -50 °C over 180 min. The shelf temperature was raised to -12 °C over 70 min after holding at -45 °C for 60 min. Then the temperature was lowered to -50 °C again over 70 min after holding at -12 °C for 360 min.

Samples were in -50 °C for 60 min. For primary drying, the shelf temperature was raised to - 10 °C for 40 min and held for 1500 min keeping the chamber vacuum at 50 mTorr. For secondary drying, the shelf temperature was raised to 25 °C over 350 min and held for 720 min. Then the temperature was lowered to 5 °C for 140 min. After all cycles are finished, stopper was placed on the vial inside the drying chamber.

**Table 1. Lyophilization procedure using Virtis lyophilizer.**

| ***Thermal Treatment*** | | | |
|---|---|---|---|
| **Step** | **Rate/Hold** | **Temperature** | **Time** |
| 1 | Hold | 4°C | 60min |
| 2 | Rate | -50°C | 180 min |
| 3 | Hold | -50°C | 60 min |
| 4 | Rate | -12°C | 70 min |
| 5 | Hold | -12°C | 360 min |
| 6 | Rate | -50°C | 70 min |
| 7 | Hold | -50°C | 60min |

| ***Primary Drying*** | | | |
|---|---|---|---|
| **Step** | **Rate/Hold** | **Temperature** | **Time** |
| 1 | Rate | -10°C | 40 min |
| 2 | Hold | -10°C | 1500 min |

| ***Secondary Drying*** | | | |
|---|---|---|---|
| **Step** | **Rate/Hold** | **Temperature** | **Time** |
| 1 | Rate | 25°C | 350 min |
| 2 | Hold | 25°C | 720 min |
| 3 | Hold | 5°C | 20 min |
| 4 | Hold | 5°C | 120 min |

### 5. Analytical Methods

### 5.1. 9G8A method

### 5.1.1. 9G8A method 1

The assay was analyzed using Bioveris instrument up to 12 months. Samples were diluted to 0.4 µg/mL with diluent (1 % BSA and 0.1 % PS-80 in PBS) using serial dilution. Standard curve dilution was also prepared with the diluent. 10µL of each sample was loaded into 96-well plate in quadruple. Then, the plate was incubated at room temperature in dark for an hour. 50 µL of TAG-labeled 9G8A antibody (5 µL of 9G8A per 3mL of diluent) was added to each well, and the plate was incubated for another hour. 50 µL of biotin-goat polyclonal antibody (5 µL of biotin-goat per 3mL of diluent) was added to the wells and incubated for another hour. 25 µL of streptavidin-beads was added to the wells and incubated for 30 minutes. Last, 115 µL of 1x-PBS was added to the wells. The plated was analyzed using Bioveris M-8 Analyzer. ECL (Enzymatic-chemo-luminescent) value was reported, and ECL value was converted to percent relative denaturation.

### 5.1.2. 9G8A method 2

Since BioVeris company could not support the instrumentation we had to develop manual method. Samples were analyzed using the new 9G8A method from time 18 month. Diluted 9G8A antibody solution (100 µL) was added to each of the wells required for analysis set. The plate was incubated for an hour at room temperature. Then, the plate was washed three times with diluent (1%BSA and 0.1% PS-80 in PBS) and blotted dry between washes. 100 µL of diluted standard, control, or samples was transferred to each of the wells. After one hour of incubation, liquid from the plate was decanted and washed three times. Secondary antibody solution was added and incubated to another hour. Liquid from the plate was decanted and washed again. 100 µL of HRP was added to each of the wells, and incubated for another 30 minutes. The liquid was decanted from the plate and washed 3 times with diluent. 100 µL of the prepared substrate reagent to each well was added, and the plate was placed on the plate reader for the analysis.

### 5.2. Size-exclusion chromatography (SE-HPLC)

Size exclusion chromatography was used as a stability indicating assay. Separations were performed with an Agilent 1100 HPLC system equipped with Chromeleon software. The method employed two columns (TosoHaas TSK gel G3000SWxl, 7.8 mm × 300 mm) attached in series along with a guard column (TSKgel Guard Column SWXL 6.0 mm × 4.0 cm). 10µg of protein was loaded onto the column and eluted isocratically at a flow rate of 0.5 mL/min with a mobile phase consisting of 20 mM Sodium Phosphate, 140 mM Sodium Chloride, pH 6.2. The protein was monitored using UV detection at 215 nm and 280 nm. Percent peak area against total area count in the chromatogram were used to quantify the amounts of high molecular weight species (HMW) and low molecular weight species (LMW).

### 5.3. Percent Oxidation

Methionine 54 oxidation detection was performed via a modified peptide map analysis. Removal of the sugars from samples required filtration (Millipore Microcon Centrifugal Filter

Devices Ultracel YM-10) via centrifugation (12000 rpm for 20 minutes at 25 °C). Sample volume was brought back up with water to the original concentration for trypsin digest. Samples were diluted to 20 µg/mL with dilution buffer (20 mM sodium phosphate, 140 mM sodium chloride, 0.005% polysorbate 80, pH 6.2). Digestion buffer (10 mM Methionine in 500 mM Tris-HCl, pH 8) was added in a 1:10 ratio of digestion buffer to total volume. Trypsin (1 mg/mL) was then added in a 1:5 ratio of trypsin to final protein concentration (in µg). Samples were incubated at 29 °C for 15 hours and then quenched with 10 µL of 25% TFA.

Oxidation detection employed a Reversed phase chromatography analysis where 2 µg (100 µL) of sample loaded onto a C8 column (Phenomenex Develosil 5u 300C8-HG 300A 150 × 2.0 mm) was separated using a step gradient at a flow rate of 2 mL/minute. (Mobile phase A consisting of 40% acetonitrile and 0.1% TFA was run for 5 minutes. Next, mobile phase B consisting of 46% actetonitrile and 0.1% TFA was ramped from 0% to 100% in 0.15 minutes and run for 15 minutes. Finally, mobile phase A ran for 10 minutes.) Column temperature was set at 55 °C. Chromatography analysis occurred on a Beckman HPLC Gold System coupled to a Thermo LCQ Deca Mass Spectrometer for ion quantification. Peak detection was at 214 nm (UV) and mass spectrometry analysis (single ion mode) used a zoom scan on selected m/z: 1264 (unoxidized), 1272 (oxidized), 1342 (partial unoxidized) and 1350 (partial oxidized). Data was reported as % oxidation.

### 5.4. Non-reduced reverse phase

Non-reduced Reverse phase high performance liquid chromatography (NRRP-HPLC) was used to determine protein content and purity, as degradation products are readily detected with this technique. Non-Reduced Reverse Phase (NRRP) HPLC was used to separate clipped species from the full-length erythropoietin monomer. Measurements were performed with a Shimadzu high performance liquid chromatography using a Phenomenex Jupiter 5µm, 300 A, C4-bonded phase silica column (150 × 4.6 mm). Mobile phase A was 0.0651% trifluoroacetic acid (v/v) in water, and mobile phase B was 0.0651% trifluoroacetic acid in 90% acetonitrile. The samples were analyzed by a gradient system from 20% B to 100% B in 135 min at a flow rate of 1.0 mL/min with detection at 215 nm. The overlay of chromatogram was used to compare the detection of degradation or clip species.

### 5.5. pH

The sample was monitored for pH using Mettler Toledo MP200 pH meter. The instrument was calibrated using pH 4 and pH 7 standard buffers.

### 5.6. Osmolarity

Osmolarity data was used to determine the percentage of reconstitution buffer.

Osmolarity was measured with an Advanced Instruments, Inc Micro Osmometer, model 330. 290 mOsm standard was used as a reference.

### 6. Results and discussion

### 6.1. 9G8A

Conformational similarity of the product samples was assessed using a 9G8A antibody binding assay (Elliott, Chang et al. 1996, Elliott, Lorenzini et al. 1996). The 9G8A monoclonal antibody recognizes a linear epitope, ERYLL, consisting of amino acids 13-17, in both native and denatured Aranesp. These amino acids become more exposed following a conformational change in, or denaturation of Aranesp, allowing for increased 9G8A binding. The data was plotted as a ratio of reactivity of the sample to that of a Darbepoetin alfa standard. A value of 1 indicates no difference in reactivity between the sample and the standard.

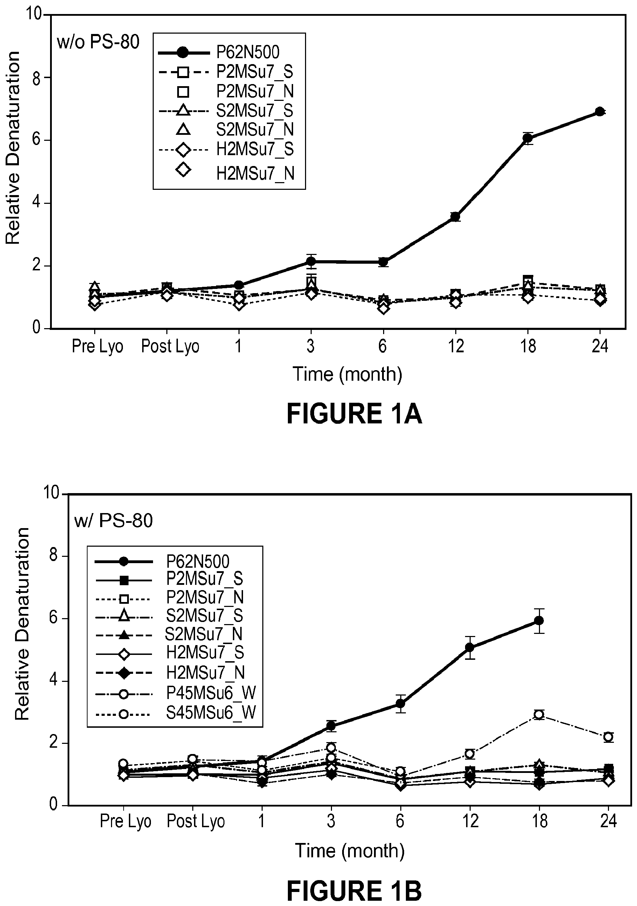

Lyophilized formulations without (A) or with (B) polysorbate-80 showed more stable than liquid formulation up to 24 month storage at 37 °C. Liquid formulation P62N showed higher relative denaturation at higher temperatures (Figure 1).

### 6.2. Aggregation detection by size exclusion chromatography (SEC)

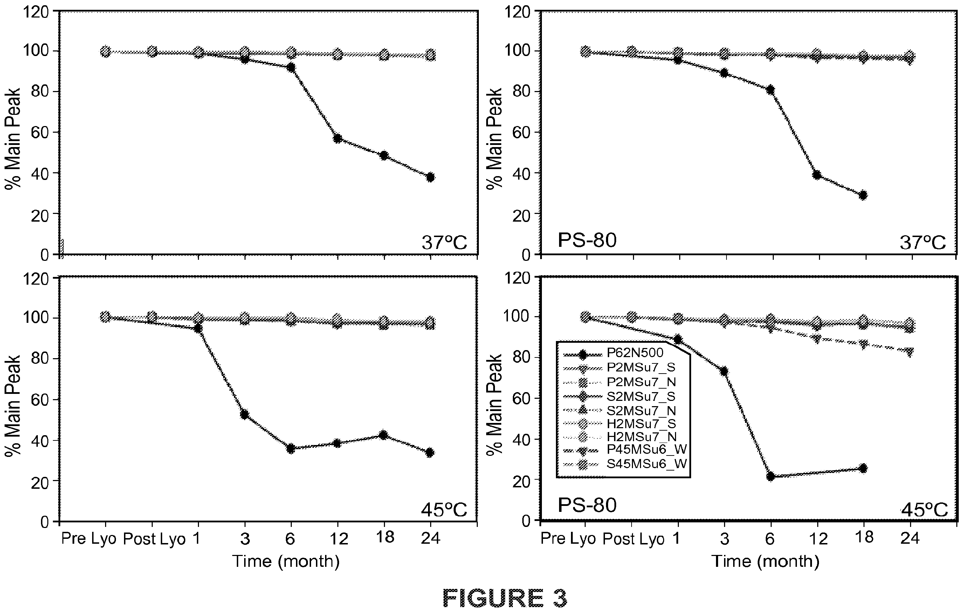

Most of Aranesp lyophilized formulations except sample in P45MSu6 demonstrated more stability than liquid formulation at higher temperatures (37 °C and 45 °C) up to 24 month storage (Figure 3). In addition, degradation is increased with temperature.

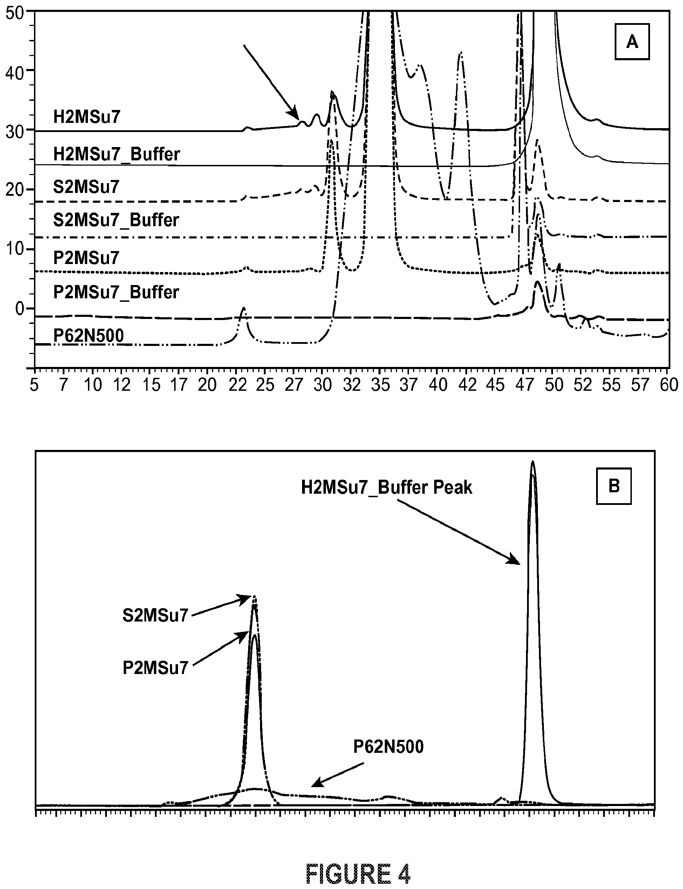

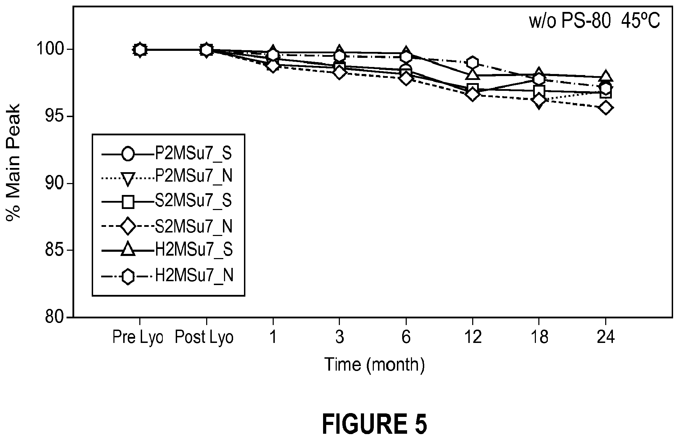

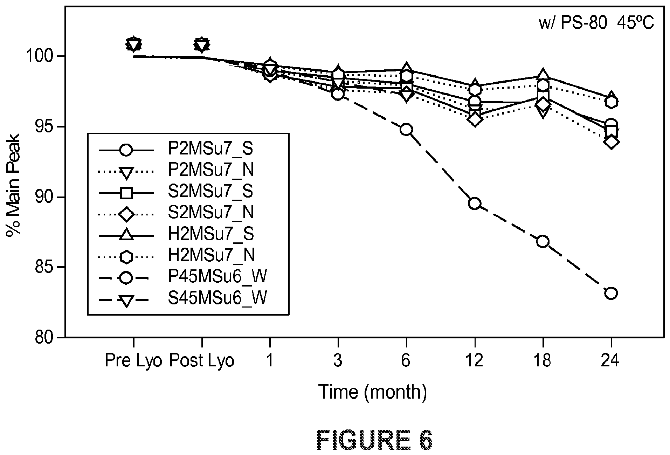

The SE-HPLC method used in this study does not separate Polysorbate peak from HMW species. Therefore, it has been observed slight increase on %HMW species for Polysorbate containing samples compared to non polysorbate-80 containing samples (Figure 3 and 6). There was no significant difference among lyophilized formulation at 4 °C and 29 °C; however, P45MSu6T was observed to have higher degradation compared to other lyophilized formulation at 37 °C, and 45 °C (Figure 5 and 6). It has been observed that H2MSu7 was more stable than other formulations (Figure 4). Sample in H2MSu7 formulation showed least dimer peak (arrow) in the overlay after stored 24 month at 45 °C. In addition, there was no significant difference between samples reconstituted with Sorbitol or sodium chloride.

### 6.3. pH measurement

There was less than 10% change in most of the formulations in all temperature (4 °C, 29 °C, 37 °C and 45°C) for 24 month storage.

### 6.4. Percent Oxidation

Methionine resides at position 54 in Aranesp and is prone to oxidation. It is this oxidation of the Trypsin digested samples that was quantified by LC/MS analysis. Data shown is % oxidation.

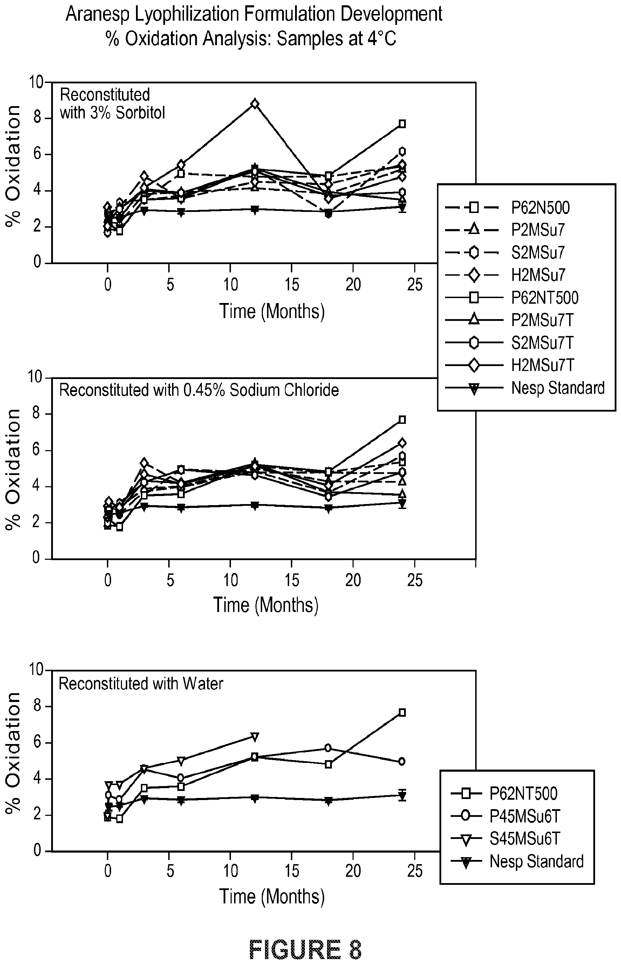

After 24 months of storage at 4 °C, all lyophilized formulations showed equal or less oxidation than the liquid formulations (Figure 8). Furthermore, no difference was observed between the three reconstitution buffers. The addition of Polysorbate-80 exhibited no significant effect on the lyophilized formulations' stability.

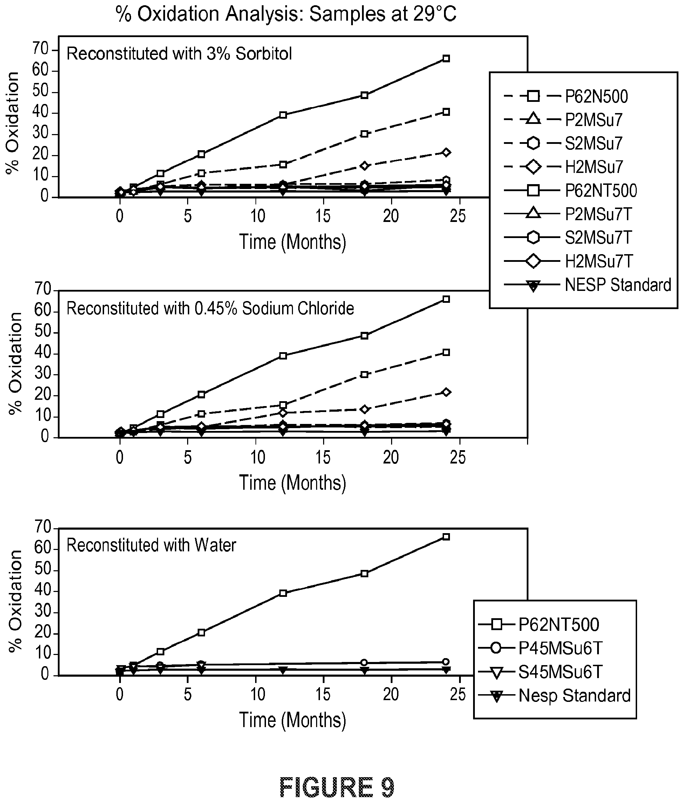

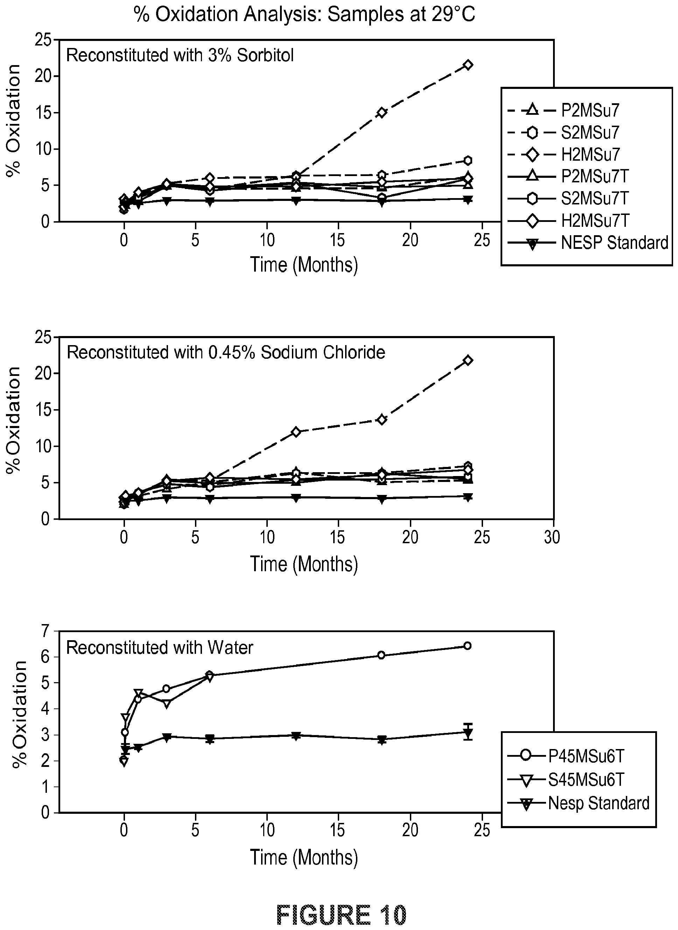

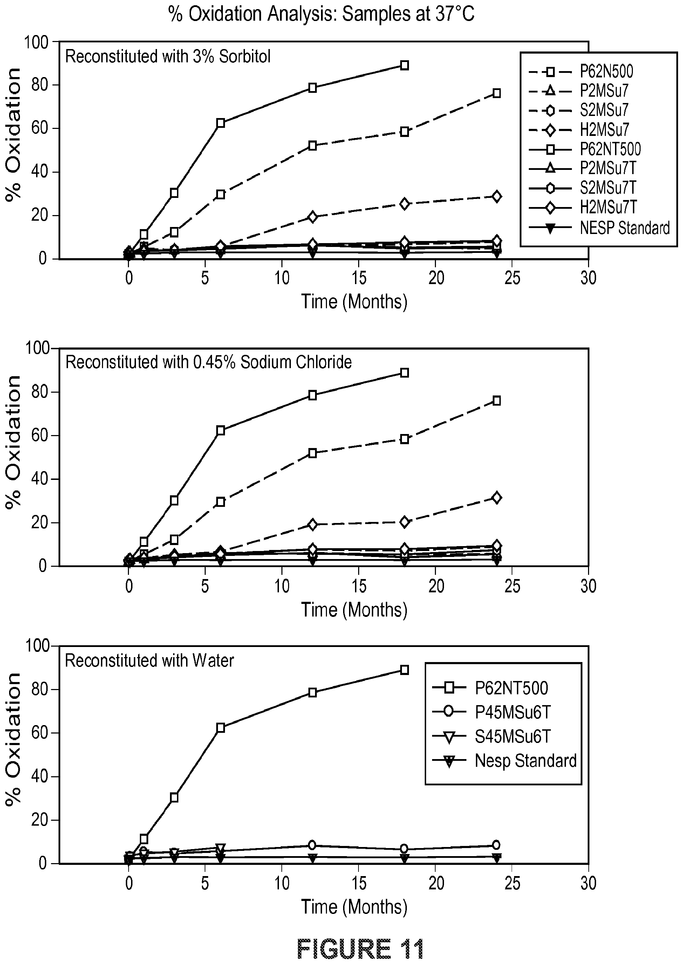

Storage at elevated temperatures (29 °C, 37 °C, and 45 °C) for up to 24 months resulted in all lyophilized formulations expressing significantly lower oxidation levels than the liquid formulations for all three reconstitution types, although oxidation increased as temperature increased (Figures 9-11). However, Histidine with no Polysorbate lyophilized formulation had significantly higher oxidation when compared to all the other lyophilized formulations by 12 months. No difference was observed between the sodium chloride and Sorbitol reconstitution buffers. Two additional lyophilized formulations, Histidine with Polysorbate and phosphate, also showed elevated oxidation when stored at 45 °C for up to 24 months (but the phosphate formulation showed the result with water reconstitution). Finally, although Polysorbate containing liquid formulation showed higher oxidation than the non-Polysorbate containing liquid formulation, no significant differences were observed in the lyophilized formulations.

### 6.5. Degradation and Clip species detection by Non-reduced reverse phase HPLC

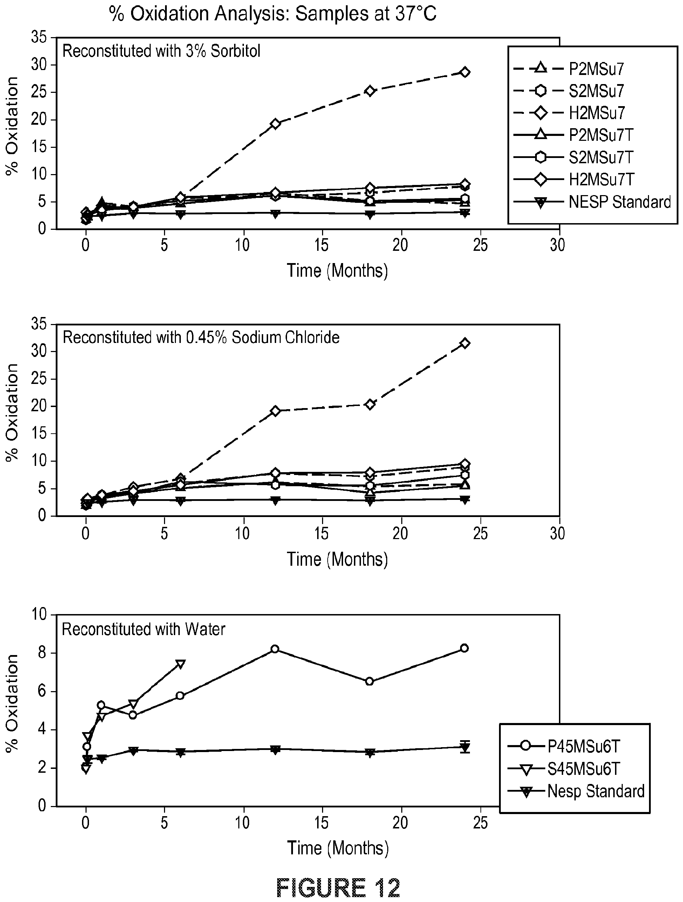

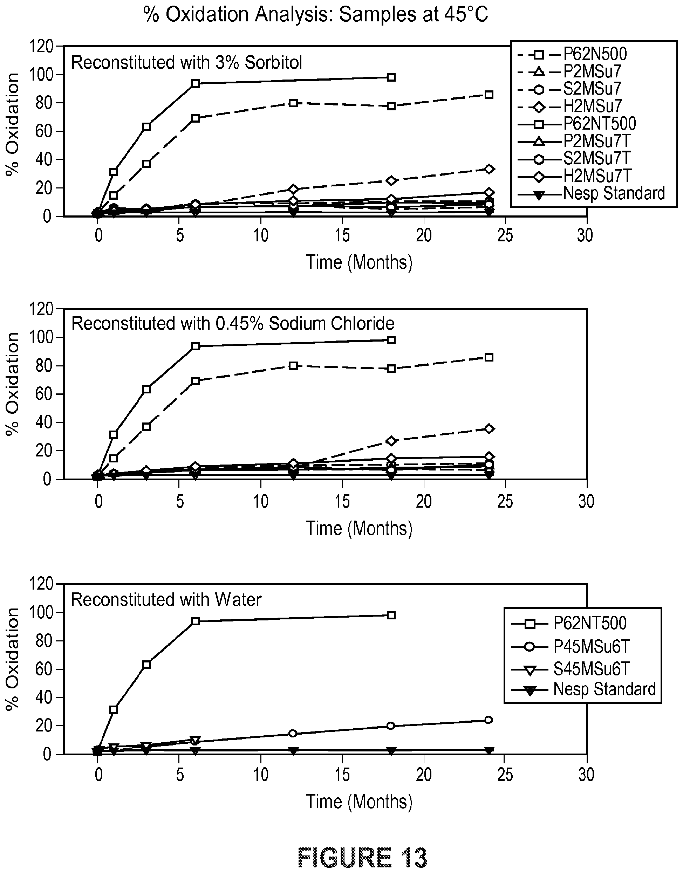

Non-reduced reverse phase HPLC is used to detect degradation and clip species. Overlays of chromatograms from non-reduced reversed phase chromatography of Aranesp^{®} after stored 24 months at 45 °C were shown in Figure 12. Figure 13 showed the overlay of chromatograms after stored at 24 month at 37 °C. The three lyophilized samples did not show any degradation or clip species with two different reconstitute diluents after stored 24 month at 4 °C, 29 °C, 37 °C and 45 °C. However, the liquid formulation sample was degraded a lot for both temperatures. The main peak of the sample in liquid formulation was almost diminished at 45 °C.

### 7. Conclusions

Lyophilized formulations of Aranesp were evaluated for stability at room temperature up to 45 °C and 24 month. Data obtained suggests that there is no significant change in attributes evaluated such as aggregates, clips, unfolding species and oxidation. Formulations containing lower Mannitol 2% showed better stability profile compared to 4.5 % Mannitol formulations. Data obtained from this feasibility suggests that Aranesp lyophilized formulation is stable at room temperature up to at least 45 °C and at least 24 months.

### Appendix 1.

### Title: Feasibility of developing room temperature stable lyophilized Aranesp formulation with LyoTip

**Purpose:** To develop and optimize NESP lyophilization formulation for LyoTip, 0.25 mL (2mg/mL) of each formulation was lyophilized and set up for stability studies at four temperatures. This sample will be diluted with 1 mL of reconstitution solution to make 500 µg/mL.
**Temperature and storage condition:** 4 °C, 29°C, 37 °C, 45 °C
**Interval:** 0, 1, 3, 6, 12, 18, 24 months
**Buffer:** sodium succinate, sodium phosphate, Histidine
**Formulated conc.:** Starting concentration: About 2mg/mL, Reconstitute sample concentration: about 500 µg/mL
**Polysorbate concentration:** 0.005 %
**ARANESP: Concentration: 2 mg/mL**
**Vials:** 3cc vial type, vials were washed and sterilized.
**Stopper:** lyophilization stopper

### Samples preparation:

### 1. Various buffers:

1. P2MSu7: 10 mM sodium phosphate, 2 % mannitol, 0.5 % sucrose, pH 7 -11)
2. S2MSu7: 10 mM sodium succinate, 2 % mannitol, 0.5 % sucrose, pH 7
3. H2MSu7: 10 mM Histidine, 2% Mannitol, 0.5% Sucrose, pH 7
4. P45MSu6: 10mM Sodium Phosphate, 4.5% Mannitol, 0.5% Sucrose, pH 6
5. S45MSu6: 10mM Sodium Succinate, 4.5% Mannitol, 0.5% Sucrose, pH 6

### 2. Lyophilization formulation:

Aranesp bulk (100 mL per formulation) was dialyzed in formulation buffer in cold room for 2 days using a Slide-A Lyser to dialyze. After dialysis, concentration was measured by UV-Vis.

### 3. Sample preparation:

To each sample, add 10% polysorbate solution in water to make 0.005% polysorbate formulation. Each sample was filtered using 0.22µm syringe filter in the sterile hood.

### a. Sample without polysorbate

Filled 0.25 mL of sample in a 3 cc vial and lyophilization stopper is used for lyophilization samples. P62N500 sample will remain as a liquid solution for control.

| | P2MSu7_2 (1.98mg/mL) | S2MSu7_2 (2.03mg/mL) | H2MSu7_2 (1.66mg/mL) | P62N500 (0.5mg/mL) |
|---|---|---|---|---|
| buffer | 0 | 0 | 0 | 18.75 |
| Dialyzed sample | 25 | 25 | 25 | 6.25 |
| total | 25 | 25 | 25 | 25 |

### b. Sample with polysorbate

| | P2MSu7T_2 (1.98mg/mL) | S2MSu7T_2 (2.03mg/mL) | H2MSu7T_2 (1.66mg/mL) | P45MSu6T_2 (2.16mg/mL) | S45MSu6T_2 (2.27mg/mL) | P62NT500 (0.5mg/mL) |
|---|---|---|---|---|---|---|
| buffer | 0 | 0 | 0 | 0 | 0 | 18.625 |
| Dialyzed sample | 24.9875 | 24.9875 | 24.9875 | 8.00 | 5 | 6.25 |
| amount of 10% polysorbate 80 | 0.0125 | 0.0125 | 0.0125 | 0.004 | 0.0025 | 0.125 |
| total | 25 | 25 | 25 | 8 | 5 | 25 |

Filled 0.25 mL of sample in a 3 cc vial and lyophilization stopper is used for lyophilization samples. P62N500 sample will be remain as a liquid solution for control.

### c. Placebo without polysorbate

| | P2MSu7_0 | S2MSu7_0 | H2MSu7_0 |
|---|---|---|---|
| Buffer | 25 | 25 | 25 |

### d. Placebo with polysorbate

| | P2MSu7T_0 | S2MSu7T_0 | H2MSu7T_0 | P45MSu6T_0 | S45MSu6T_0 |
|---|---|---|---|---|---|
| Buffer | 24.9875 | 24.9875 | 24.9875 | 9.995 | 9.995 |
| amount of 10% polysorbate 80 | 0.0125 | 0.0125 | 0.0125 | 0.005 | 0.005 |
| total | 25 | 25 | 25 | 10 | 10 |

### 4. Sample Description

| | | | |
|---|---|---|---|
| #1 | P2MSu7_0 | Placebo | 10 mM Sodium Phosphate, 2% Mannitol, 0.5% Sucrose, pH 7 |
| #2 | S2MSu7_0 | Placebo | 10 mM Sodium Succinate, 2% Mannitol, 0.5% Sucrose, pH 7 |
| #3 | H2MSu7_0 | Placebo | 10 mM Histidine, 2% Mannitol, 0.5% Sucrose, pH 7 |
| #4* | P45MSu6_0 | Placebo | 10mM Sodium Phosphate, 4.5% Mannitol, 0.5% Sucrose, pH 6 |
| #5* | S45MSu6_0 | Placebo | 10mM Sodium Succinate, 4.5% Mannitol, 0.5% Sucrose, pH 6 |
| #6 | P2MSu7T_0 | Placebo | 0.005% PS 80, 10 mM Sodium Phosphate, 2% Mannitol, 0.5% Sucrose, pH 7 |
| #7 | S2MSu7T_0 | Placebo | 0.005% PS 80, 10 mM Sodium Succinate, 2% Mannitol, 0.5% Sucrose, pH 7 |
| #8 | H2MSu7T_0 | Placebo | 0.005% PS 80, 10 mM Histidine, 2% Mannitol, 0.5% Sucrose, pH 7 |
| #9 | P45MSu6T_0 | Placebo | 10 mM Histidine, 2% Mannitol, 0.5% Sucrose, pH 7 |
| #10 | S45MSu6T_0 | Placebo | 10mM Sodium Phosphate, 4.5% Mannitol, 0.5% Sucrose, pH 6 |
| #11** | P62N500 | 0.5 mg/mL Liquid | 20 mM Sodium Phosphate, 140mM sodium Chloride, pH 6.2 |
| #12 | P2MSu7_2 | 1.98mg/mL | 10 mM Sodium Phosphate, 2% Mannitol, 0.5% Sucrose, pH 7 |
| #13 | S2MSu7_2 | 2.03mg/mL | 10 mM Sodium Succinate, 2% Mannitol, 0.5% Sucrose, pH 7 |
| #14 | H2MSu7_2 | 1.66mg/mL | 10 mM Histidine, 2% Mannitol, 0.5% Sucrose, pH 7 |
| #15* | P45MSu6_2 | 2.16mg/mL | 10 mM Histidine, 2% Mannitol, 0.5% Sucrose, pH 7 |
| #16* | S45MSu6_2 | 2.27mg/mL | 10mM Sodium Phosphate, 4.5% Mannitol, 0.5% Sucrose, pH 6 |
| #17** | P62NT500 | 0.5 mg/mL Liquid | 0.005% PS80, 20 mM Sodium Phosphate, 140mM sodium Chloride, pH 6.2 |
| #18 | P2MSu7T_2 | 1.98mg/mL | 0.005% PS 80, 10 mM Sodium Phosphate, 2% Mannitol, 0.5% Sucrose, pH 7 |
| #19 | S2MSu7T_2 | 2.03mg/mL | 0.005% PS 80, 10 mM Sodium Succinate, 2% Mannitol, 0.5% Sucrose, pH 7 |
| #20 | H2MSu7T_2 | 1.66mg/mL | 0.005% PS 80, 10 mM Histidine, 2% Mannitol, 0.5% Sucrose, pH 7 |
| #21 | P45MSu6T_2 | 2.16mg/mL | 10 mM Histidine, 2% Mannitol, 0.5% Sucrose, pH 7 |
| #22 | S45MSu6T_2 | 2.27mg/mL | 10mM Sodium Phosphate, 4.5% Mannitol, 0.5% Sucrose, pH 6 |

| | | | |
|---|---|---|---|
| ^{∗}Not enough samples for these. ^{∗∗}Liquid formulation for control | | | |

### 5. Constitution of sample for analysis: Reconstituted the sample with 1 mL of following solution at each time point

a. Water for P45MSu6 and S45MSu6
   For P2MSu7, S2MSu7and H2MSu7, use following solution
b. 3% Sorbitol: 1mL
c. 0.45% sodium chloride: 1mL

### Analysis and Time points:

1 mL per sample vial

| **Time Point** | **Temp** |
|---|---|
| Time Zero | T=0, Pre-Lyo, Post-Lyo |
| 1 month | 4 °C, 29°C, 37 °C, 45 °C |
| 3 month | 4 °C, 29°C, 37 °C, 45 °C |
| 6 month | 4 °C, 29°C, 37 °C, 45 °C |
| 12 month | 4 °C, 29°C, 37 °C, 45 °C |
| 18 month | 4 °C, 29°C, 37 °C, 45 °C |
| 24 month | 4 °C, 29°C, 37 °C, 45 °C |

### Analytical Method:

C4 Jupiter column 300A; 250 × 4.6 mm; S./No.: 202394, batch no. 5267-4
Mobile phase; A-0.065% TFA in water, B-0.065 % TFA in 95% Acetonitrile,
Run time: 145 min, Flow rate 0.75 ml/min,
Detection: 215 nm, 230 nm, 280 nm
Gradient: 20% B (5min); 20% - 70 % B (100 min); wash and equilibration
(40 min)
Sample load: 3 µg

### SEC HPLC:

Column: Two columns of TSK G3000swxl, Flow rate 0.5 mL/min isocratic, Run time 80 min, Buffer 100 mM sodium phosphate, 0.5 M sodium chloride at pH 6.9
Detection: 215nm, 230 nm, 280 nm, Injection for Buffer: 100µL,
HSA and Tween sample: 3 µg.
Report at 215 nm for total area and % main peak of total area.

### Sample List per time point

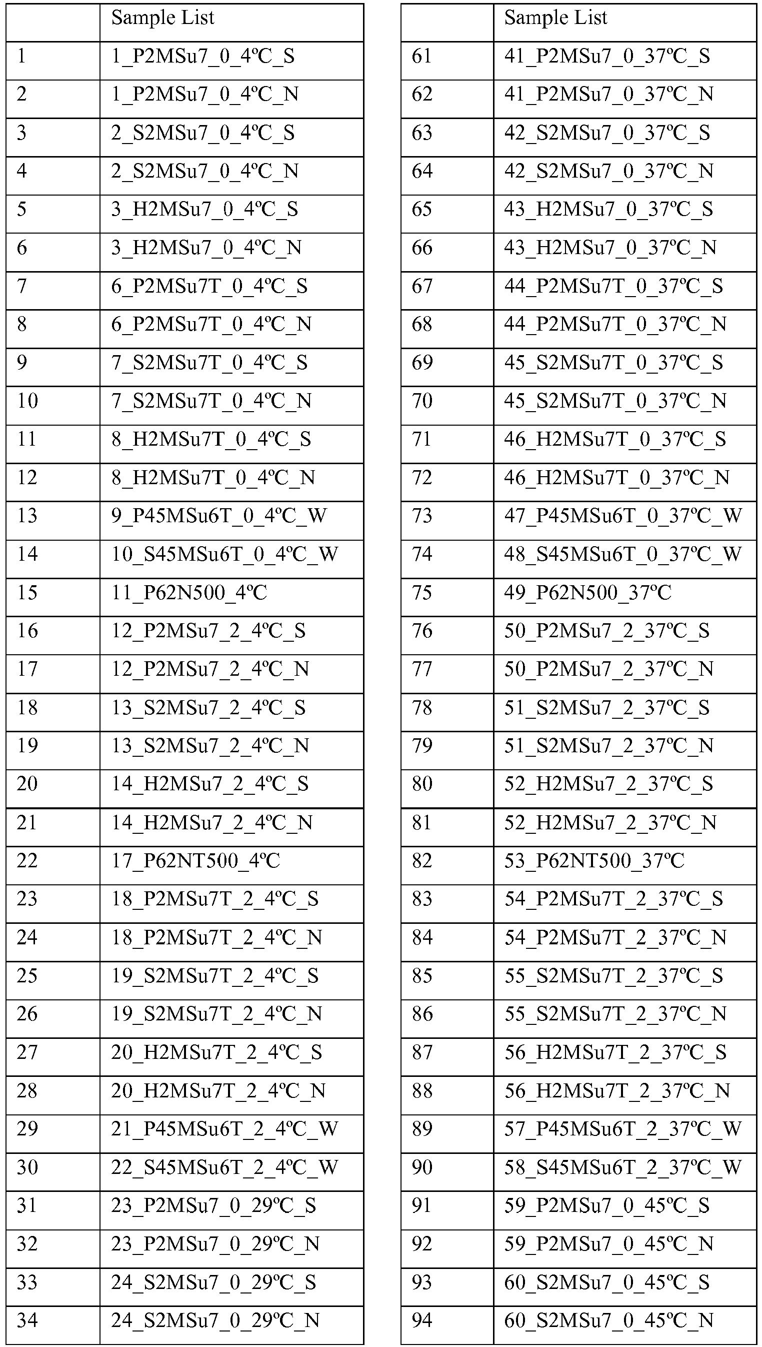

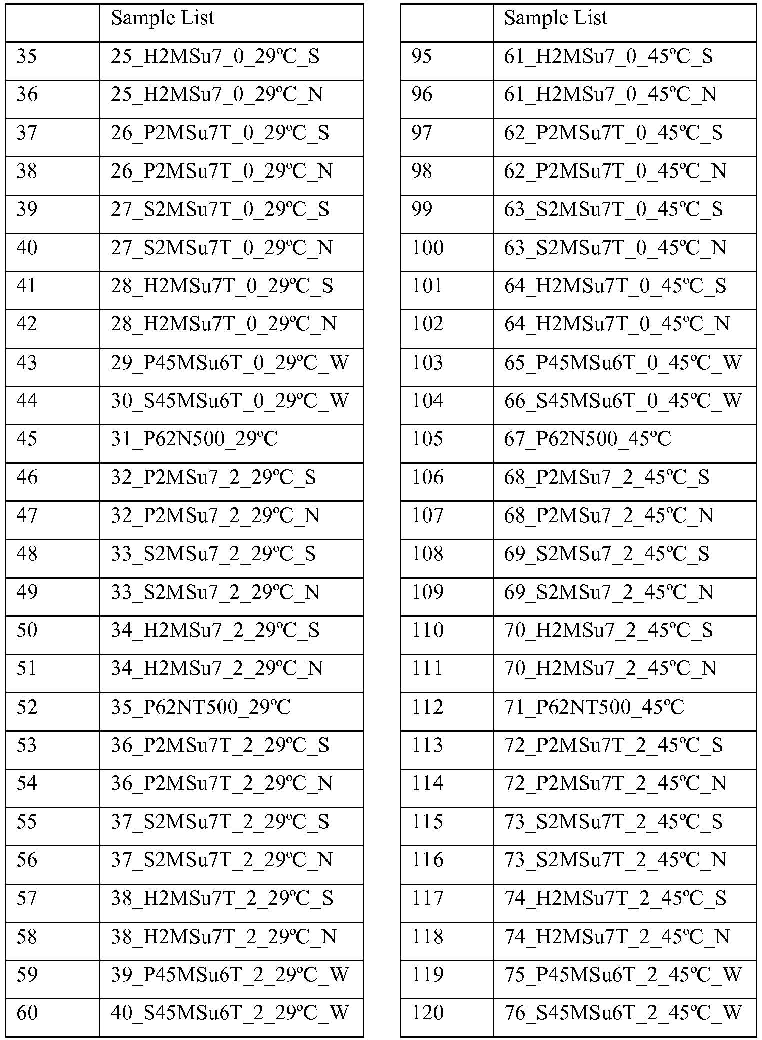

### EXAMPLE 2

### 1. Purpose

An Aranesp lyophillization formulation was selected from the above Example 1, the compatibility study of CZ plastic vial (Diakyo Crystal Zenith) against glass vial was needed because the LyoTip^{®} is made from Crystal Zenith plastic material. In this study, the comparability of the container (Daikyo Crystal Zenith) to glass vial on effect of Aranesp^{®} shelf stability and possibility of Aranesp^{®} sting free formulation was conducted. For sting free formulation, 1% benzyl alcohol with 0.7% sodium chloride at pH 7.0 solution was used to reconstitute the lyophilized sample.

### 2. Summary of Experiments

Aranesp^{®} (1.84 mg/mL) was formulated in 10 mM Histidine, 2% Mannitol, 0.5% Sucrose, 0.005% polysorbate 80, pH 7 by dialysis of Aranesp formulation buffer (20 mM sodium phosphate, 140 mM sodium chloride, pH 6.2). The dialyzed sample (0.25 mL) was filled in 2cc Crystal Zenith vials and 3cc glass vials and was lyophilized. The lyophilized samples were stored at 4°C, 29°C, 37°C, and 45°C for 12 months for shelf stability. At each time point, samples were reconstituted with 1 mL of three reconstitution solutions (0.70% NaCl, pH 7; 0.70 % NaCl plus 1% Benzyl Alcohol, pH 7.0; 0.70% NaCl, pH 7 with 0.004% PS-80) to make final concentration of 500 µg/mL. The reconstituted samples were analyzed by SE-HPLC (Size Exclusion HPLC), 9G8A (Monitoring relative protein unfolding), pH, % Oxidation, RP-HPLC (impurities and clips), sub-visible particle, Concentration and Osmolarity.

### 3. Materials and Equipment

| | |
|---|---|
| 3.1. | Aranesp bulk (1.84 mg/mL) |
| 3.2. | Polysorbate-80 |
| 3.3. | Blow-back glass 3cc vials (type 1 glass EP) |
| 3.4. | Diakyo Crystal Zenith vials (DS CZ VIAL 2mL 13mm made by West. Daikyo long stoppers for lyophilization was used for stopper. |
| 3.5. | 10mM Histidine, 2% Mannitol, 0.5% Sucrose, pH 7(H2MSu7) |
| 3.6. | Slide-A Lyser dialysis cassette was obtained from Pierce. |
| 3.7. | Corning filter (0.22 µm) was used for filtration. |
| 3.8. | Samples were dialyzed for two days in the cold room |
| 3.9. | Virtis lyophilizer was used for lyophillization. |

### 4. Sample Preparation Procedure

Aranesp^{®} (1.84 mg/mL) was formulated in 10 mM Histidine, 2% Mannitol, 0.5% Sucrose, 0.005% polysorbate 80, pH 7 by dialysis of Aranesp formulation buffer (20 mM sodium phosphate, 140 mM sodium chloride, pH 6.2) in the cold room (2-8°C) for 2 days using a Slide-A Lyser dialysis cassette. The buffer was checked for pH and osmolarity at the room temperature before dialysis. After dialysis, the concentration of the sample was checked. Polysorbate-80 1% (w/v) stock solution was added to the sample to make 0.005%. The 1% polysorbate-80 solution was made by adding 0.25 g of polysorbate 80 to 25 mL of HM2MSu7 buffer. The samples were filtered using Corning filter (0.22 µm) in the sterile hood. The filtered sample (0.25 mL) was filled into two different kinds of vials (Crystal Zenith and glass vial) and was subjected to lyophilization cycle in building 8 using Virtis lyophilizer (Table 2). 0.25 mL of placebos and protein containing formulation were lyophilized and stored at 4°C, 29°C, 37°C, and 45°C for 12 months for shelf stability. At each time point, samples were reconstituted with 1 mL of three reconstitution solutions (0.70% NaCl, pH 7; 0.70 % NaCl plus 1% Benzyl Alcohol, pH 7.0; 0.70% NaCl, pH 7 with 0.004% PS-80) to make final concentration of 500 µg/mL. The reconstituted samples were analyzed for SEC-HPLC (Size Exclusion HPLC), 9G8A (Monitoring relative protein unfolding), pH, % Oxidation, Non-reduced RP-HPLC (impurities and clips), sub-visible particle, Concentration and Osmolarity. Sample list, time points, and temperature are indicated in Appendix 1 and 2.

**Table 2. Lyophilization cycle**

| ***Thermal Treatment*** | | | |
|---|---|---|---|
| **Step** | **Rate/Hold** | **Temperature** | **Time** |
| 1 | Hold | 5°C | 5 min |
| 2 | Rate | -50°C | 120 min |
| 3 | Hold | -50°C | 60 min |
| 4 | Rate | -12°C | 60 min |
| 5 | Hold | -12°C | 180 min |
| 6 | Rate | -50°C | 60 min |
| 7 | Hold | -50°C | 60min |

| ***Primary Drying*** | | | |
|---|---|---|---|
| **Step** | **Rate/Hold** | **Temperature** | **Time** |
| 1 | Hold | -50°C | 30 min |
| 2 | Rate | -15°C | 60 min |
| 3 | Hold | -15°C | 600 min |

| ***Secondary Drying*** | | | |
|---|---|---|---|
| **Step** | **Rate/Hold** | **Temperature** | **Time** |
| 1 | Rate | 25°C | 420 min |
| 2 | Hold | 25°C | 600 min |
| 3 | Hold | 25°C | 1000 min |

### 5. Analytical Method

### 5.1. 9G8A method

Samples were diluted as required from 0.34 to 4.2 µg/mL in 1x PBS, 1% BSA, 0.1% polysorbate-80 buffer, and distributed into a 96 well plate. Fifty µL of TAG (BioVeris) labeled 9G8A (2 µg/mL) anti-rHuEPO antibody was added, and the samples were incubated for 1 hour at room temperature on a plate shaker. Fifty µL of an affinity-purified, biotinylated rabbit anti-rHuEPO antibody (stock concentration 0.5 µg/mL) was then added and the samples were incubated for 1 hour at room temperature on the plate shaker. Next, 25 µL of streptavidin-coated beads (stock concentration, 0.566 µg/mL) diluted to 0.6 µg /mL in 1× phosphate-buffered solution (PBS), 1% bovine serum albumin, and 0.1% polysorbate-80 were added, and the samples were incubated for 30 minutes at room temperature on the shaker plate. The plate was transferred to an M8 analyzer (IGEN International, Gaithersburg, MD) and the chemiluminescent signal was measured according to the manufacturer's instructions. The chemiluminescent signal was converted to a ratio of sample to Aranesp bulk standard and reported as relative reactivity with 1 being equal to the standard and values >1 indicating protein denaturation.

### 5.2. Size-exclusion chromatography (SE-HPLC)

Size exclusion chromatography was used as a stability indicating assay. Separations were performed with an Agilent 1100 HPLC system equipped with Chromeleon software. The method employed two columns (TosoHaas TSK gel G3000SWxl, 7.8mmX300mm) attached in series along with a guard column (TSKgel Guard Column SwXL 6.0mm × 4.0 cm). 10µg of protein was loaded onto the column and eluted isocratically at a flow rate of 0.5mL/min with a mobile phase consisting of 20mM Sodium Phosphate, 140mM Sodium Chloride, pH 6.2. The protein was monitored using UV detection at 215nm and 280nm. Percent peak area against total area count in the chromatogram were used to quantify the amounts of high molecular weight species (HMW) and low molecular weight species (LMW).

### 5.3. Concentration

Protein concentrations were determined by UV/VIS spectroscopy using an Agilent UV/Vis spectrophotometer model 8453 system using Chemstation software. The absorption at 280nm was determined using Beer's law with an extinction coefficient of 0.98 in a cuvette with a 1cm path length.

### 5.4. Sub-visible particle analysis with HIAC

Sub-visible particle counting was monitored by HIAC in the 2-25µm range. Prior to analysis, samples were degassed under vacuum for 1 hour with open cap. Four 0.2mL measurements were made for each sample and prior to sample measurements Millipore water was used to blank the system. The first run was discarded, and the last three were averaged to obtain the cumulative counts per milliliter. Under USP guidelines, particles sizes of 2, 5, 7.5, 10, 15, 20, and 25 µm were monitored.

### 5.5. % Oxidation

Oxidation detection assay was performed including sample digestion by trypsin, followed by reversed phase chromatography with mass spectrometry detection (RP-HPLC/MS). The LC/MS oxidation assay was used to determine the percentage of oxidized methionine 54. Solutions of all samples were prepared at 500 µl or 10 µg of 0.02 mg/mL sample in 20 mM Sodium Phosphate, 140 mM Sodium Chloride, 0.005% Polysorbate 80, at pH 6.2. Then 50 µl of 0.5 M Tris-HCl, 10 mM Methionine, at pH 8.0 was added to the sample. Trypsin (2 µl of 1 mg/ml) was added to sample and incubated overnight at 29 °C. Sample was quenched with 10 µl of 25% TFA. Analysis of the samples was achieved using a Beckman HPLC System Gold (Osiris, System ID # 408774) coupled to a LCQ Deca Mass Spectrometer (Promasseous). The flow rate was 0.20 ml/min using isocratic method with 42% Acetonitrile, 0.1% TFA as a mobile phase, Zorbax 300SB-C8 column (150 × 2.1 mm, 5 µm, 300 Angstrom, Part Number: 883750-906) at 60 °C temperature. The detection was done at 215 and 280 nm with UV and used zoom scan on selected m/z: 1264 (unoxidized), 1272 (oxidized), 1342 (partial unoxidized) and 1350 (partial oxidized). The percentage of oxidized product was evaluated using the ratios of specific peptide peaks.

### 5.6. Non-reduced reverse phase

Non-reduced Reverse phase high performance liquid chromatography (NRRP-HPLC) was used to determine protein content and purity, as degradation products are readily detected with this technique. Non-Reduced Reverse Phase (NRRP) HPLC was used to separate clipped species from the full-length erythropoietin monomer. Measurements were performed with a Shimadzu high performance liquid chromatograph using a Phenomenex Jupiter 5µm, 300 A, C4-bonded phase silica column (150 × 4.6 mm, 00F-4167-E0). Mobile phase A was 0.0651% trifluoroacetic acid (v/v) in water, and mobile phase B was 0.0651% trifluoroacetic acid in 90% acetonitrile. The samples were analyzed by a gradient system from 20% B to 100% B in 135 min at a flow rate of 1.0 mL/min with detection at 215 nm. The overlay of chromatogram was used to compare the detection of degradation or clip species.

### 5.7. pH Measurement

The sample was monitored for pH using Mettler Toledo MP200 pH meter. The instrument was calibrated using pH 4 and pH 7 standard buffers.

### 5.8. Osmolarity Determination

Osmolarity was measured with an Advanced Instruments, Inc Micro Osmometer, model 330. 290 mOsm standard was used as a reference.

### 6. Results and Discussion

### 6.1. 9G8A

Conformational similarity of the product samples was assessed using a 9G8A antibody binding assay (Elliott, Chang et al. 1996, Elliott, Lorenzini et al. 1996). The 9G8A monoclonal antibody recognizes a linear epitope, ERYLL, consisting of amino acids 13-17, in both native and denatured Aranesp. These amino acids become more exposed following a conformational change in, or denaturation of Aranesp, allowing for increased 9G8A binding. The data was plotted as a ratio of reactivity of the sample to that of a Darbepoetin alfa standard. A value of 1 indicates no difference in reactivity between the sample and the standard.

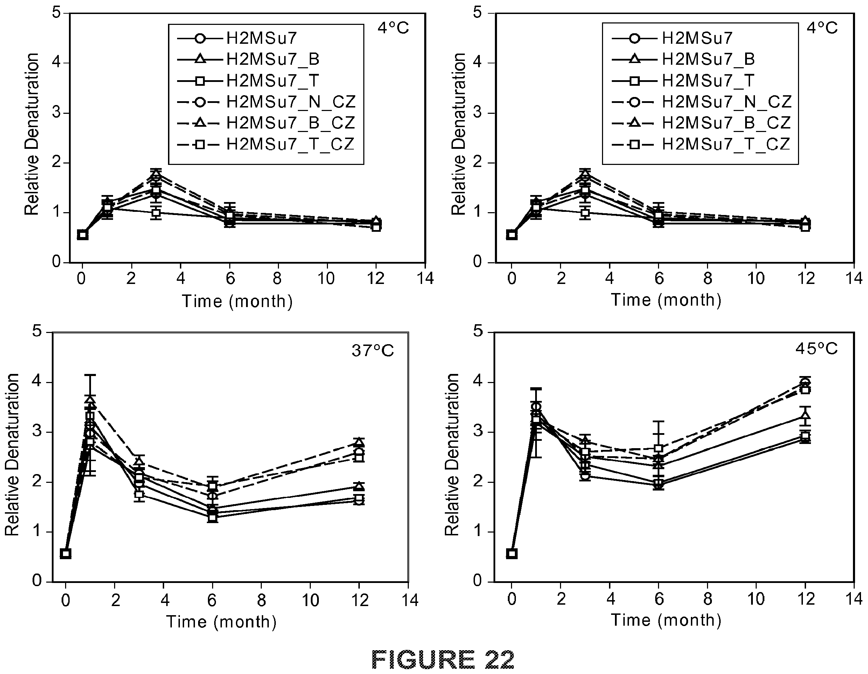

There was no significant difference when compared between different reconstitution diluents including benzyl alcohol and polysorbate-80. However, there was a slight increase in relative denaturation at higher temperature for samples in Crystal Zenith (CZ) vial when it was compared to the samples in glass vial. Results are summarized in Figure 22.

### 6.2. Aggregation detection by size exclusion chromatography (SEC)

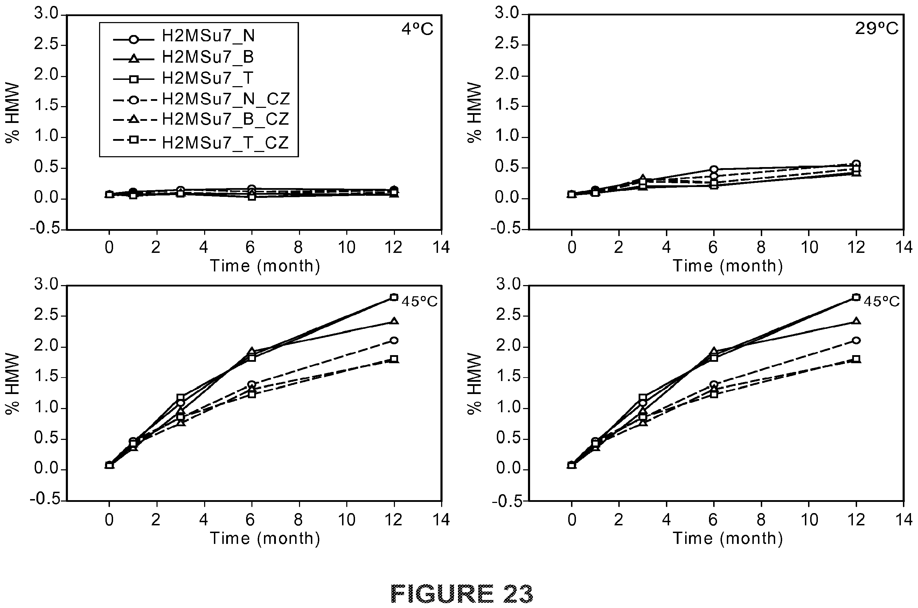

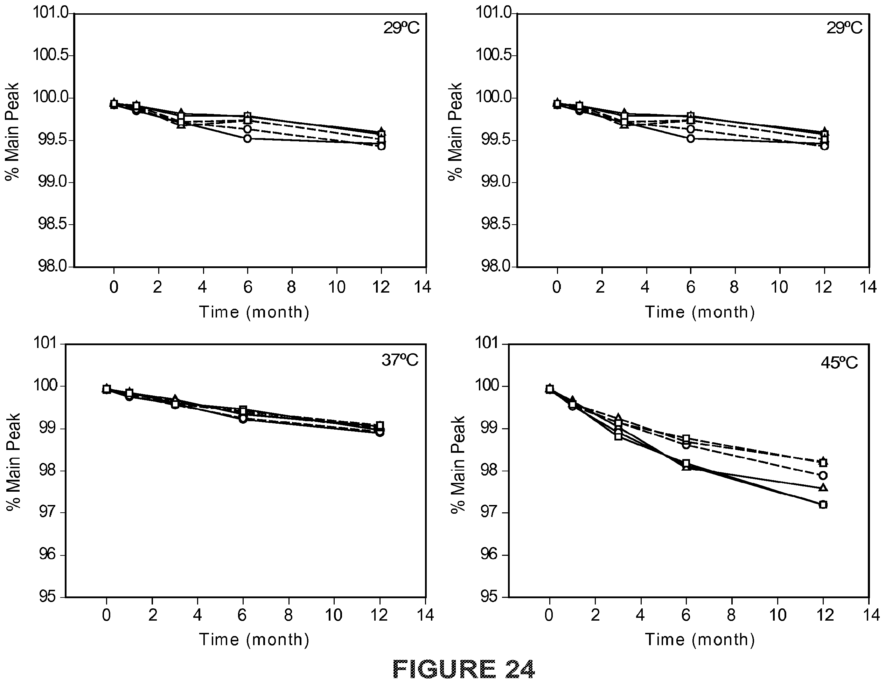

There was no significant difference in % HMW species (Figure 23) in Aranesp^{®} when compared three different reconstitution diluents including benzyl alcohol and polysorbate-80 for up to 37 °C. However, there was a slight increase in high molecular weight species at 45 °C for samples in glass vial when it was compared to the samples in Crystal Zenith (CZ) vial (Figure 23). The Aranesp^{®} in CZ vial showed higher % main peak than the samples in the glass vial at 45 °C (Figure 24).

### 6.3. Sub-visible particle analysis,

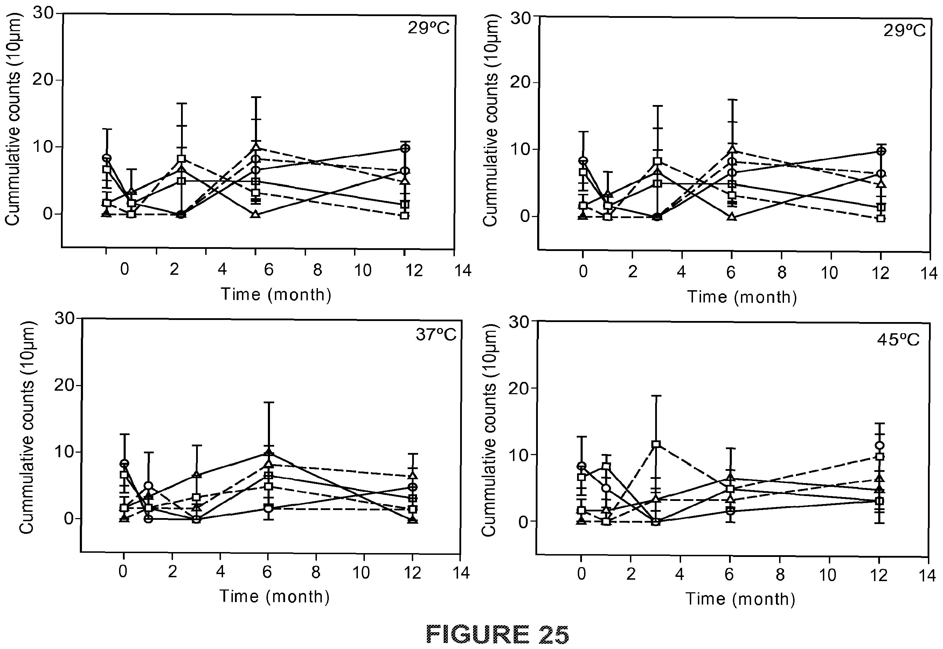

No trend in particle count was observed between samples in glass and cz vials. The particle counts stayed below the USP guidelines for 10 µm and 25 µm particles in both protein and placebo samples at all four temperatures (Figure 25). The USP guide line is ≤6,000 particles per container for particles ≥10µm and ≤600 particles per container for particles ≥25µm.

### 6.4. Visual Inspection

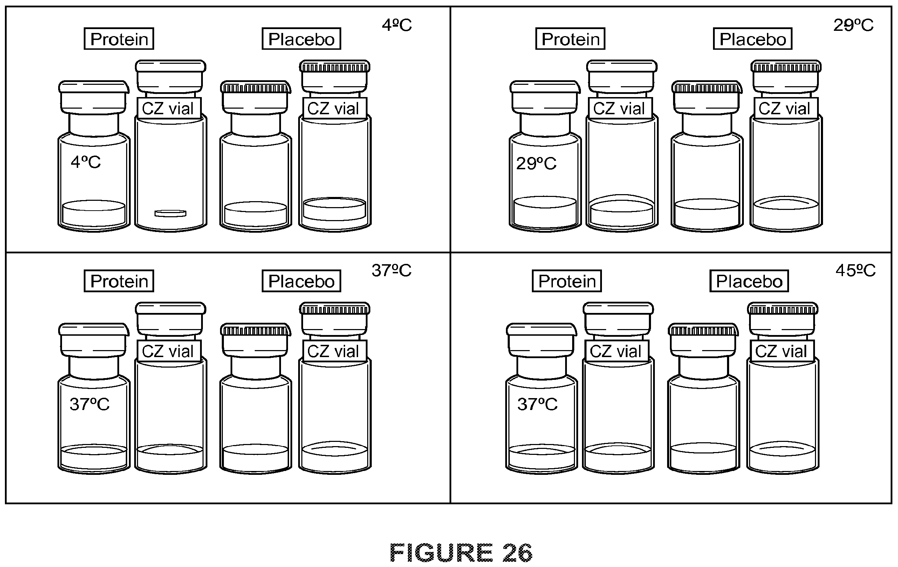

Samples in CZ vial stored at 4 °C for 12 month showed the lyophilized cake melted away for both protein and placebo in CZ vial (Figure 26). It shows moisture penetrate into CZ vial.

### 6.5. Concentration Measurement

Protein concentration was decreased slightly after 12 month storage at 4 °C samples in CZ vial. However, in other temperatures and time points, there were no difference in protein concentration between samples and containers.

### 6.6. % Oxidation

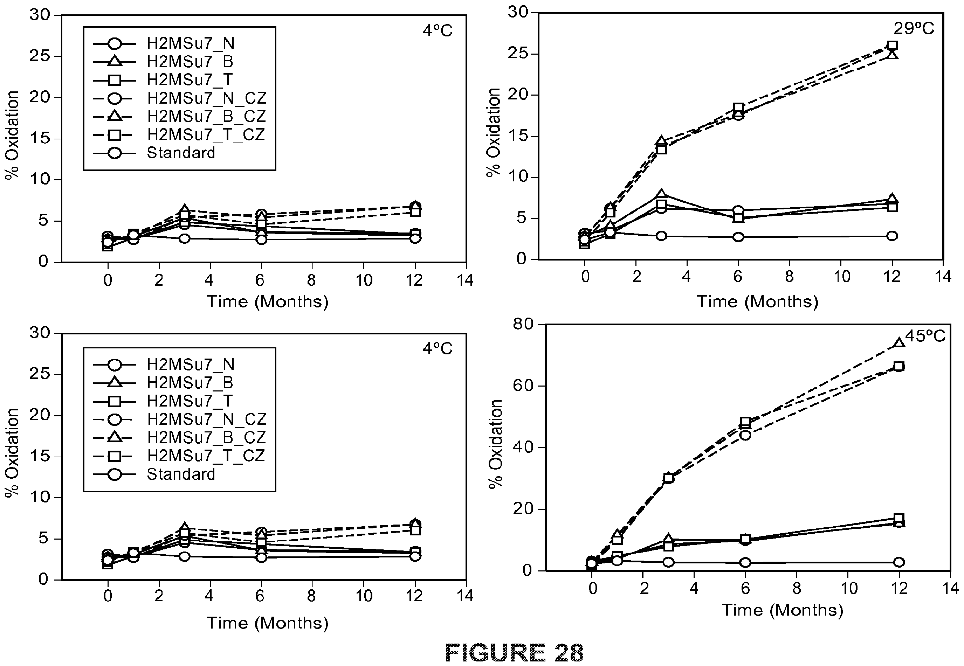

The % oxidation for Aranesp stored for 12 months at four different temperatures (4 °C, 29 °C, 37 °C and 45 °C) is shown in Figure 28. The faster rate of oxidation observed for the samples in CZ vial as compared to the samples in glass vial at 29 °C, 37 °C and 45 °C. After 12 month incubation, samples in CZ vial showed about 25 % oxidation while samples in glass vial showed about 5% oxidation at 29 °C storage temperature. The difference is more significant for higher temperature storage. There were no differences among three reconstitution buffers (0.7% sodium chloride, 1% benzyl alcohol or 0.004% polysorbate-80) for four temperatures (4 °C, 29 °C, 37 °C and 45 °C) up to 12 month storage.

### 6.7. Degradation and Clip species detection by Non-reduced reverse phase HPLC

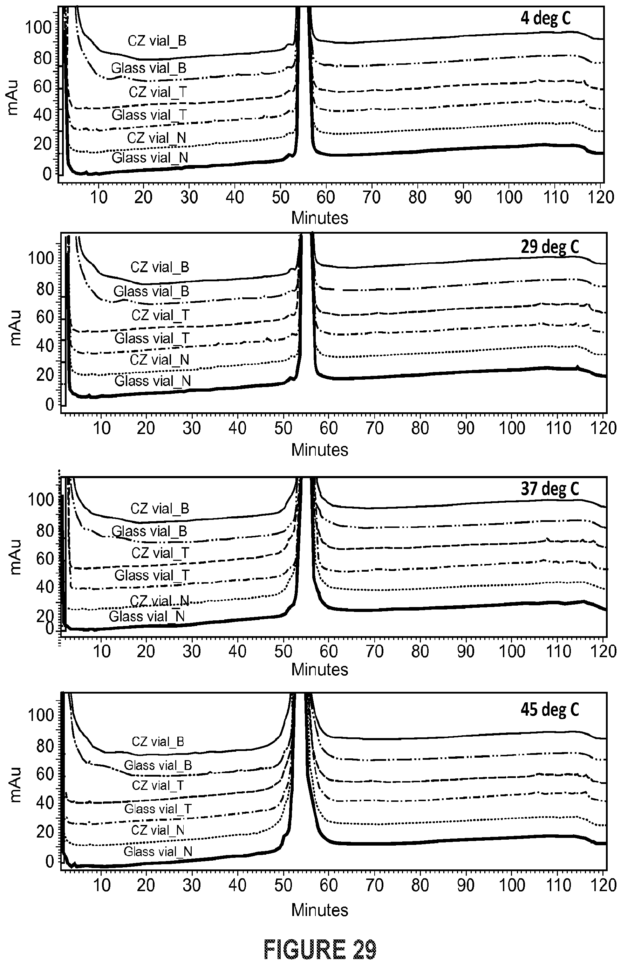

Non-reduced reverse phase HPLC was used to detect degradation and clip species. Overlays of chromatograms from non-reduced reversed phase chromatography of Aranesp^{®} after stored 12 months at four different temperatures were shown in Figure 29. The lyophilized samples in CZ vial or in glass vial did not show any degradation or clip species with three different reconstitute diluents after storage for 12 month at 4 °C, 29 °C, 37 °C and 45 °C.

### 6.8. pH measurement

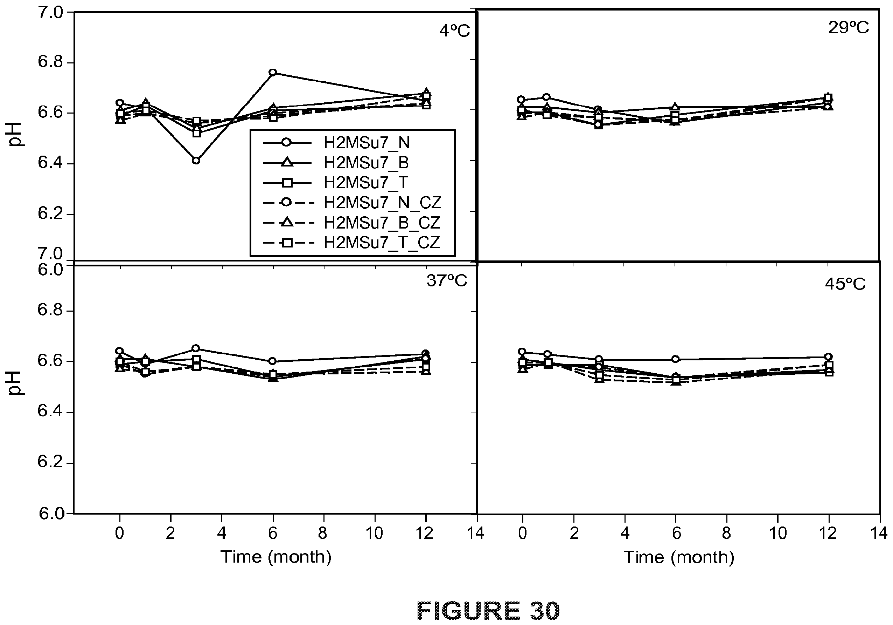

The initial pH did not changed over the storage time and at four different temperatures (Figure 30).

### 6.9. Osmolarity measurement

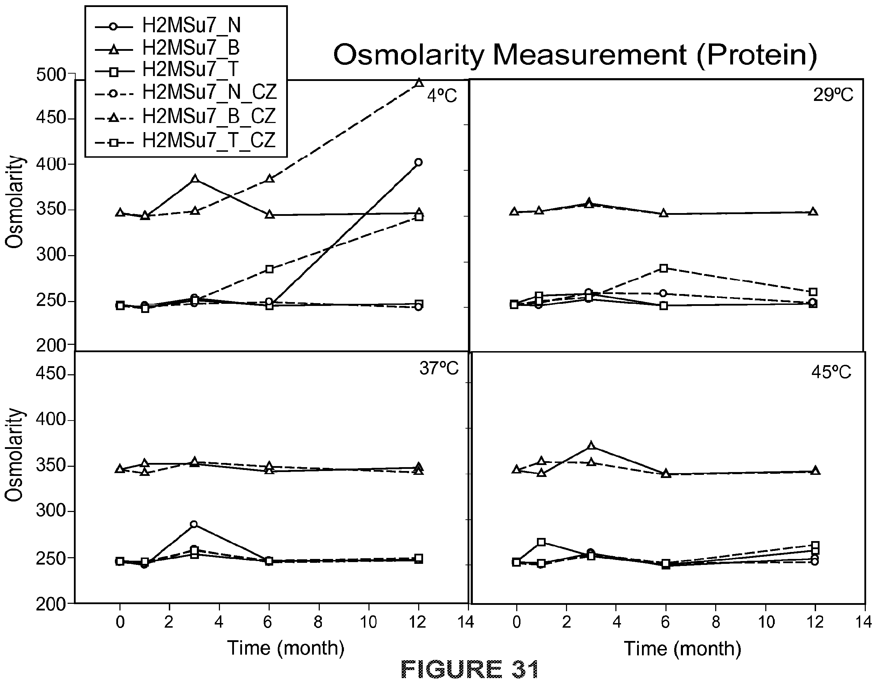

The samples reconstituted with benzyl alcohol shows higher osmolarity than other samples for all four temperature. There is no difference between samples in different containers. Even though there is outlier at 4 °C samples but there is no trend. Results are summarized in Figure 31.

### 7. Conclusions

We investigated potential reformulation of Aranesp that can be stored at room temperature for at least 2 years and can be sting free.

Using the Aranesp lyophilized formulation (10 mM Histidine, 2% Mannitol, 0.5% Sucrose, 0.005% polysorbate 80, pH 7) and three reconstitution buffers (0.7% sodium chloride, 1% benzyl alcohol, 0.004% polysorbate-80), a preliminary container comparative study was conducted for Aranesp stability in Daikyo Crystal Zenith (CZ) vial against glass vial. Physical and chemical degradation was monitored using SE-HPLC, % oxidation, pH, RP-HPLC and 9G8A immunoassay. The result shows no difference between reconstitution buffers (0.7% sodium chloride, 1% benzyl alcohol, 0.004% polysorbate-80), and different containers (CZ and glass vial) based on 9G8A (unfolding), sub-visible particle, RP-HPLC (clips) and SE-HPLC (aggregation) analyses. However, a decrease of cake structure and higher percent oxidation was observed for samples in CZ vial compared to samples in glass vial. This showed the CZ vial was susceptible for moisture and oxygen penetration. This study showed that 1% benzyl alcohol can be used as reconstitution buffer for sting free purpose. Also, lyophilization Aranesp formulation at pH 7 can reduced the sting effect that can be stored at room temperature for at least 2 years.

### 8. Appendix

### Appendix 1. Sample List

| **Sample name** | **Vial** | **Reconstitute buffer** | **Protein Concentration** |
|---|---|---|---|
| 1_H2MSu7_N_500_4C | Regular | 0.7% NaCl | 500 µg/mL |
| 2_H2MSu7_B_500_4C | Regular | 0.7% NaCl, 1% Benzyl alcohol | 500 µg/mL |
| 3_H2MSu7_T_500_4C | Regular | 0.7% NaCl, 0.004% Polysorbate | 500 µg/mL |
| 4_H2MSu7_N_500_CZ_4C | Diakyo Crystal Zenith | 0.7% NaCl | 500 µg/mL |
| 5_H2MSu7_B_500_CZ_4C | Diakyo Crystal Zenith | 0.7% NaCl, 1% Benzyl alcohol | 500 µg/mL |
| 6_H2MSu7_T_500_CZ_4C | Diakyo Crystal Zenith | 0.7% NaCl, 0.004% Polysorbate | 500 µg/mL |
| 7_H2MSu7_N_0_4C | Regular | 0.7% NaCl | 0 µg/mL |
| 8_H2MSu7_B_0_4C | Regular | 0.7% NaCl, 1% Benzyl alcohol | 0 µg/mL |
| 9_H2MSu7_T_0_4C | Regular | 0.7% NaCl, 0.004% Polysorbate | 0 µg/mL |
| 10_H2MSu7_N_0_CZ_4C | Diakyo Crystal Zenith | 0.7% NaCl | 0 µg/mL |
| 11_H2MSu7_B_0_CZ_4C | Diakyo Crystal Zenith | 0.7% NaCl, 1% Benzyl alcohol | 0 µg/mL |
| 12_H2MSu7_T_0_CZ_4C | Diakyo Crystal Zenith | 0.7% NaCl, 0.004% Polysorbate | 0 µg/mL |

**Appendix 2.** Time points and dates.

| **Time point** | **Temp** |
|---|---|
| 0 month | 4°C |
| 1 month | 4°C, 29°C, 37°C, 45°C |
| 3 month | 4°C, 29°C, 37°C, 45°C |
| 6 month | 4°C, 29°C, 37°C, 45°C |
| 12 month | 4°C, 29°C, 37°C, 45°C |

### Example 3

### Summary

A lyophilized formulation of anti-EPO monoclonal antibody was developed for room temperature stable formulation. Two lyophilized formulations and two concentrations (100 ug/mL, 500 ug/mL) were investigated.
1. GMST:10mM Na Glutamate (from Glutamic acid), 4% mannitol, 2% sucrose, 0.01% polysorbate 20, pH 5.2
2. HMST:10mM Histidine, 4% mannitol, 2% sucrose, 0.01% polysorbate 20, pH 6.0 Samples in histidine formulation showed slightly higher % main peak than in glutamate buffer based on SE-HPLC results. Overall, samples from two formulations did not show any significant changes in aggregation and overall conformation and thermal stability after incubation at 37°C for up to 12 weeks.

### Materials, Methods, and Equipment

### Materials:

### Anti-EPO mAb 8C10 (see U.S. Patent Appl. Publication No. 20130295113A1, application number 13/888,777)

Formulation buffer:
- GMST:10mM Na Glutamate (from Glutamic acid), 4% mannitol, 2% sucrose, 0.01% polysorbate 20, pH 5.2
- HMST:10mM Histidine, 4% mannitol, 2% sucrose, 0.01% polysorbate 20, pH 6.0

**Table 3. EPO mAb Sample Code and description**

| Sample Code | Sample Description | Concentration |
|---|---|---|
| 01 EMGMST0 | Placebo-GMST | 0 |
| 02 EMHMST0 | Placebo-HMST | 0 |
| 03 EMGMST100 | anti-EPO mAb 8C10-GMST | 100 mcg/mL |
| 04 EMHMST100 | anti-EPO mAb 8C10-HMST | 100 mcg/mL |
| 05 EMGMST500 | anti-EPO mAb 8C10-GMST | 500 mcg/mL |
| 06 EMHMST500 | anti-EPO mAb 8C10-HMST | 500 mcg/mL |

### Methods:

### Size Exclusion Chromatography (SEC)

Size Exclusion (SEC) method was used for monitoring the monomer and high molecular weight species (HMWS), such as dimer and aggregation. The method employed two columns (Tosoh G3000SWxl, 7.8mmX300mm) attached in series along with a guard column (TSKgel Guard Column SWXL 6.0mmx4.0cm) using an Agilent 1100 (Name: KGB) HPLC. The flow rate was 0.5 mL/min isocratic with a mobile phase consisting of 50mM Sodium Phosphate; 300mM Sodium Chloride, pH 6.8. The detection of protein was monitored using UV detection at 215nm. Percent area count against total area count was utilized for % main peak and % HMW (High Molecular Weight) peak.

### Reduced CE-SDS method

The CE-SDS method was conducted using Beckman Coulter PA-800 (two-faces) with 100 x 800 aperture and a bare fused silica, 50um ID at 20cm (effective length) and /30cm ( total length) with Beckman SDS-MW kit. The data was collected using Beckman Karat 32 software. Peak integrations were performed using Chromeleon, and Excel was used for %purity calculation based on the corrected area. 1.0 mL of 100 mcg/mL samples of GMST 100 and HMST 100, and 200 uL of 500 mcg/mL of GMST 500 and HMST 500 were each concentrated to final volume of 45 uL using 30K MWCO Nanospin. 50uL of samples buffer and 5uL of 2-mercaptoethanol were added and heated at 70°C for 10 minutes; then loaded to PCR sample tube for CE-SDS assay.

### DSC

The thermal stability of the samples was assessed by DSC on a MicroCal VP-Capillary DSC system in which temperature differences between the reference and sample cell are continuously measured, and calibrated to power units. This data channel is referred to as the DP signal, or the differential power between the reference and sample cell. The unfolding of the protein molecules appears as an endothermic transition on the DSC thermogram and can be characterized by the thermal transition (melting) temperatures (Tₘ). The samples were heated from 4°C to 110°C at a heating rate of 60 °C/hour. The pre-scan time was 15 minutes, and the filtering period was 10 seconds. The concentrations used in the DSC experiments were 0.1 and 0.5 mg/mL. The data analysis was done using MicroCal Origin 7 software.

### Analytical ultracentrifugation (AUC) analysis

The samples were analyzed by a Beckman Coulter ProteomeLab XL-I instrument. The anti EPO mAb samples were stored at 4 °C before the analysis and analyzed without dilution. The sedimentation velocity experiments were performed at 45,000 rpm following the absorbance at 280 nm. Experiments were performed in double-sector centerpiece cell assemblies with quartz windows. Scans were collected at 20 °C without delay between scans, 120 scans per each sample. The AUC-SV data were analyzed using Sedfit v11.8 using the `Continuous c(s) distribution' model. The parameters for c(s) distribution analyses were: s range 2 to 30, resolution 200, and data selection for analysis 6.4 to 6.8. In the AUC-SV analysis frictional ratio, time invariant noise and meniscus position were allowed to float during the non-linear least squares fit. The following parameter was used for the AUC-SV distribution analysis: partial specific volume 0.73 mL/g, density and viscosity of both Glu and His buffers were 1.02100 g/mL, and 0.01210 Pa.

### Sample Preparation Method

Sample was dialyzed against two formulation buffer at 2-8°C for 3 days. The concentration of sample was measured using A280 nm after dialyzed sample. Samples were diluted or concentrated to 100 µg/mL and 500 µg/mL with two formulation buffers. Samples were filtered with 0.22 µm filter, 1 mL of sample was aliquoted into a 3cc vial, and vials were stoppered. Some sample was saved for pre-lyo time zero testing. Samples were lyophilized according to lyophilized process. After lyophilization, samples were stored in a cold room until for each time points. Samples were reconstituted with 1 mL water at each time point.

### Lyophilization procedure

Sample vials were loaded on 'pre-chilled' (4°C) shelves of a lyophilizer. Samples were subjected for three steps of freezing, primary drying and secondary drying. First the samples were hold for 30 minutes at 4 °C; cooled down from 4 °C to -45 °C over 123 min, and held at 45°C for 180min. The shelf temperature was raised to -12 °C over 150 min after holding at -45 °C for 180 min. Then the temperature was lowered to -45 °C again over 150 min after holding at -12 °C for 240 min. Samples were in -45 °C for 120 min. For primary drying, the shelf temperature was raised to -10°C and hold for 25 hours keeping the chamber vacuum at 120 mTorr after made the condenser temperature less than -50 °C. For secondary drying, the shelf temperature was raised to 25 °C over 234 min while lowering chamber pressure to 100 mTorr. After that the shelf temperature was at 25 °C for 11 hours while keeping the chamber pressure at 100 mTorr. After all cycles are finished, stopper was placed on the vial inside the drying chamber.

### Results and Discussion

### Size exclusion chromatography (SEC)

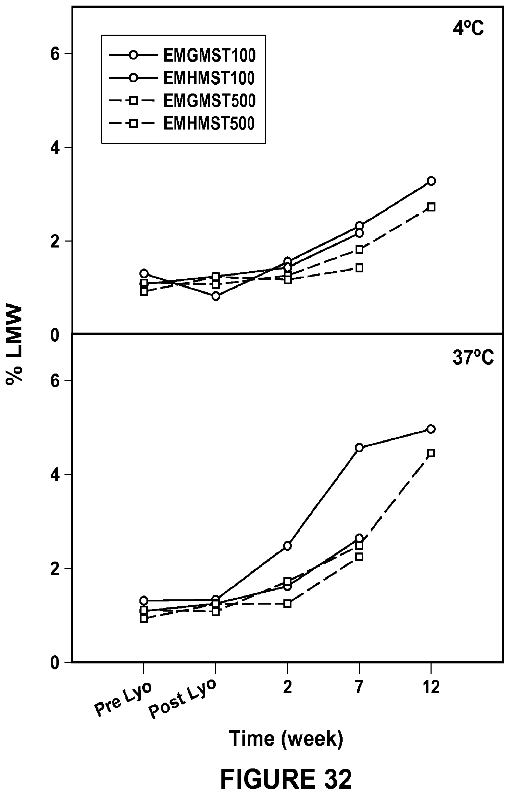

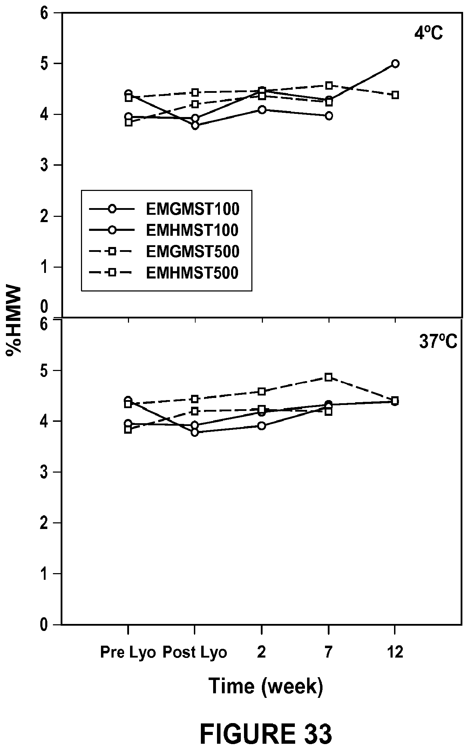

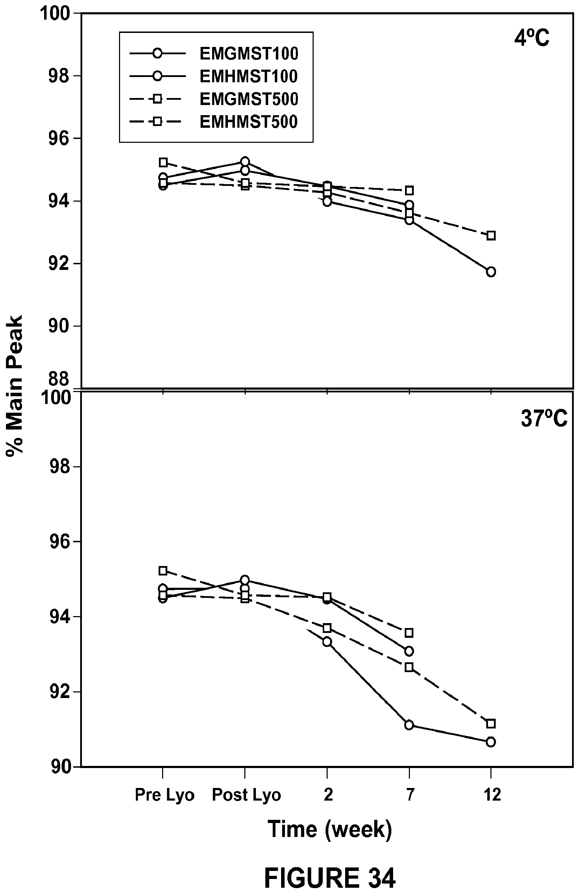

Both lyophilized samples showed slight increasing of % low molecular weight species (%LMW) up to 12 week storage for both 4°C and 37°C storage (Figure 32). Based in 7 week data at higher temperature (37°C), sample in histidine formulation showed less %LMW for 100 µg/mL. Also for % high molecular weight species (%HMW), the histidine formulation showed slightly less than the sample in glutamate formulation at higher temperature (Figure 33). However, there was no difference before and after lyophilization. Overall, the sample in histidine formulation showed higher % main peak than in glutamate formulation (Figure 34). Histidine formulation was not assessed at 12 weeks due to the availability of the samples

### Degradation by Capillary Electrophoresis (CE-SDS)

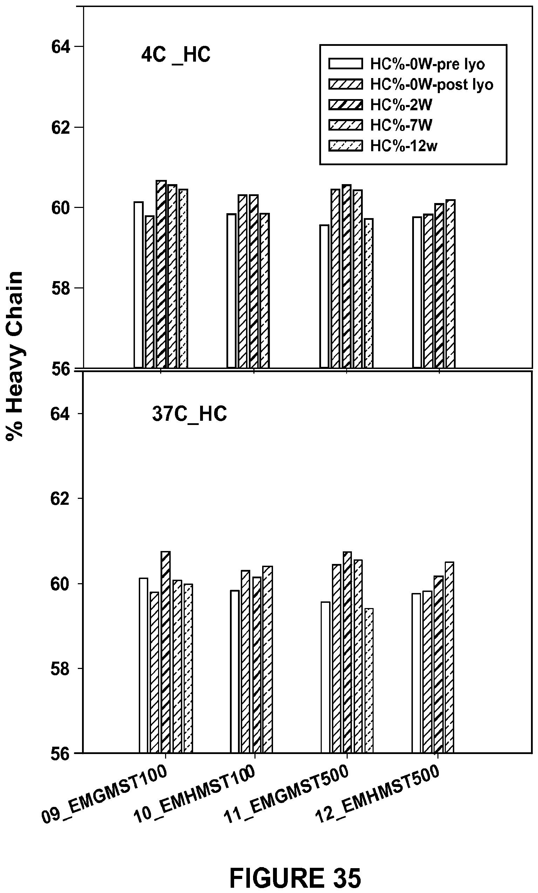

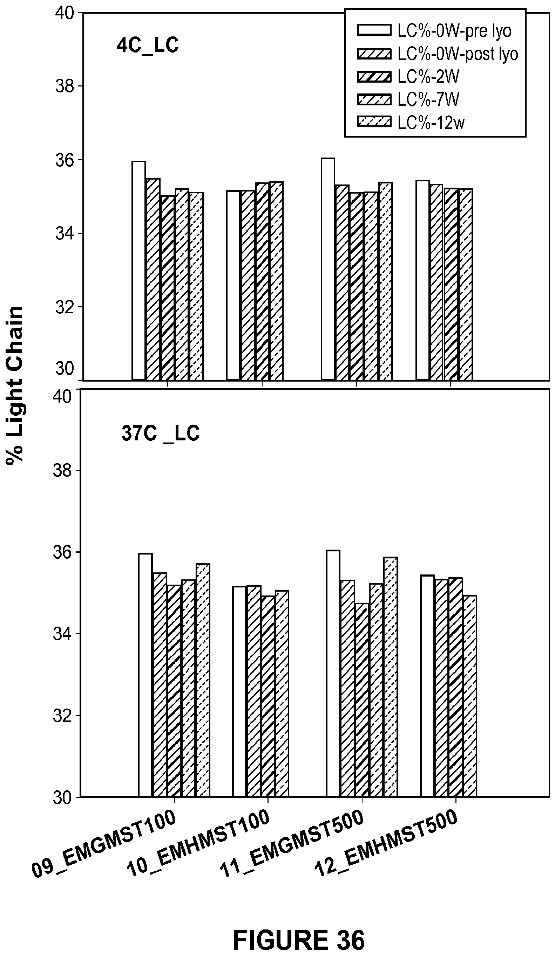

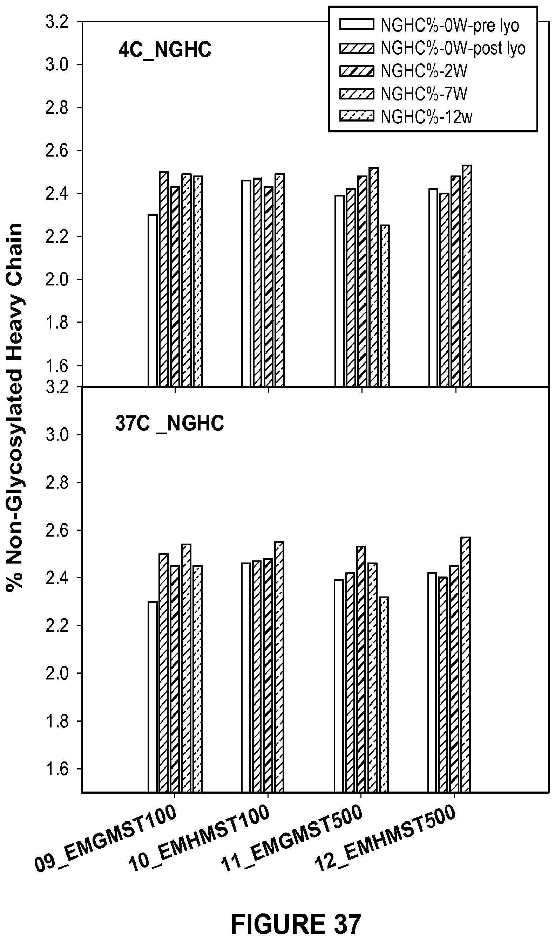

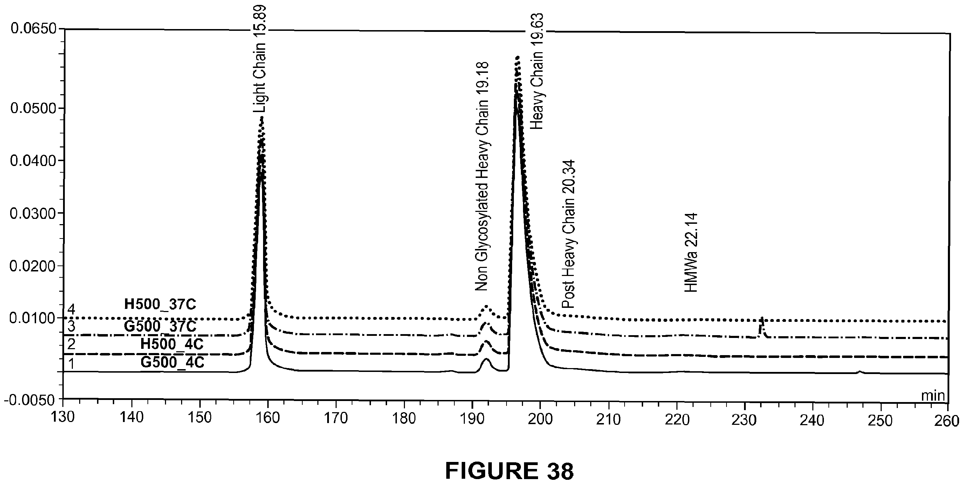

This analytical method is used to analyze the Heavy chain (HC), Light chain (LC), Non- glycosylated HC (NGHC), and other minor peak species under reducing and denaturing conditions. Reduced CE-SDS separates proteins based on the differences in their hydrodynamics size under reducing and denaturing conditions. The protein species are bound to SDS, an anionic detergent and electrokinetically injected into a bare fused silica capillary filled with SDS gel buffer. An electrical voltage is applied across the capillary, under which the SDS coated proteins are separated by their difference in migration in a hydrophilic polymer based solution. Proteins are detected by a photodiode array (PDA) detector as they pass through a UV detection window. Purity is evaluated by determining the percent corrected peak area of each component. There was no significant difference between formulations for % heavy chain (Figure 35), % light chain (Figure 36) and % non-glycosylated heavy chain (Figure 37) by reduced CE-SDS method. The overlaid electropherograms shown in Figure 38 demonstrated that there were no detectable degradation species observed in all the conditions tested at 7 weeks.

### Thermal stability by differential scanning calorimetry (DSC)

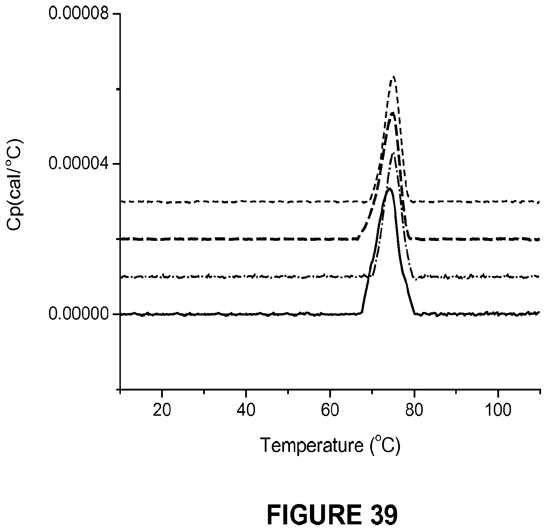

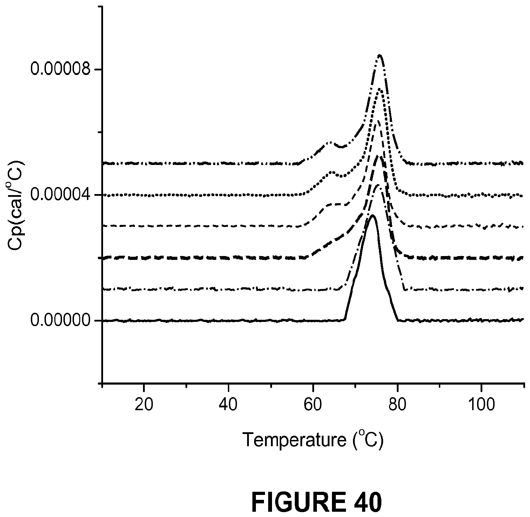

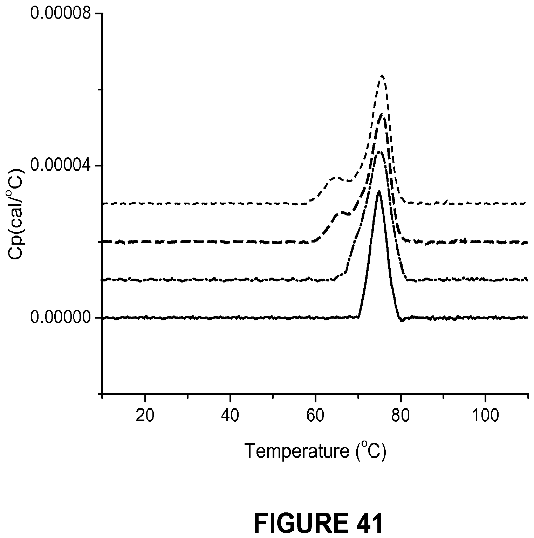

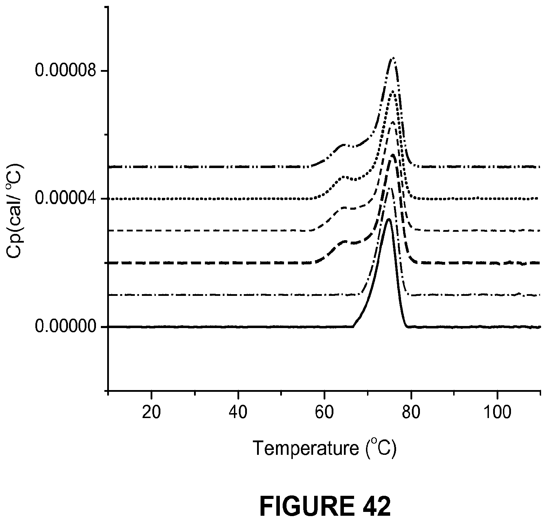

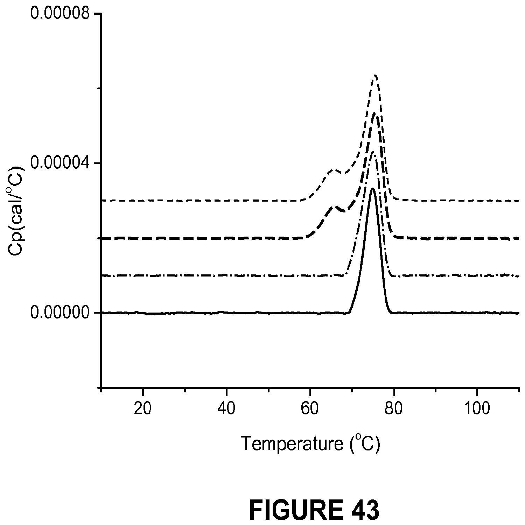

Differential Scanning Calorimetry (DSC) was used to analyze the anti-EPO mAb samples in two different buffers. With this technique the relative thermal stability of each sample can be compared. The DSC scans of the 4 pre-lyo samples in GMST and HMST buffers are shown in Figure 39 and the thermal transition temperatures are listed in Table 3. The typical standard deviation of the thermal transition temperature measurements was within ± 0.5°C. The DSC data suggested that the thermal stability of the pre-lyo samples was similar in both buffers and at both concentrations. The comparison of DSC scans of each anti-EPO mAb sample under different conditions and time points are shown in Figures 40-43 and the thermal transition temperatures are also listed in Table 4. Once again, the DSC data suggested that the thermal stability of the samples remain similar in both buffers and at both concentrations and temperatures with time for up to 12 weeks. It appeared that the C_{H}2 domain of each sample after 7 weeks unfolds at lower temperature compared to that at time zero. Further studies suggested that the differences could be caused by analyzing the samples at different time.

**Table 4. Thermal transition temperatures of each sample**

| | | | |
|---|---|---|---|
| | | | **C_{H}2/Fab/C_{H}3** |
| Pre-lyo (T=0) | 03_EMGMST100 | | 74.1 |
| | 04_EMHMST100 | | 75.0 |
| | 05_EMGMST500 | | 74.9 |
| | 06_EMHMST500 | | 74.9 |
| Lyo (T=0) | 03_EMGMST100 | | 75.3 |
| | 04_EMHMST100 | | 75.3 |
| | 05_EMGMST500 | | 75.2 |
| | 06 EMHMST500 | | 75.0 |

| | | **C_{H}2** | **Fab/C_{H}3** |
|---|---|---|---|
| 4°C (T=7 weeks) | 03_EMGMST100 | 63.6 | 75.6 |
| | 04_EMHMST100 | 66.4 | 75.6 |
| | 05_EMGMST500 | 64.7 | 75.8 |
| | 06_EMHMST500 | 65.9 | 75.6 |
| 37 °C (T=7 weeks) | 09_EMGMST100 | 64.8 | 75.4 |
| | 10_EMHMST100 | 64.8 | 75.8 |
| | 11_EMGMST500 | 64.5 | 75.8 |
| | 12_EMHMST500 | 65.8 | 75.5 |
| 4°C (T=12 weeks) | 03_EMGMST100 | 64.7 | 75.9 |
| | 05_EMGMST500 | 64.6 | 75.7 |
| 37°C (T=12 weeks) | 09_EMGMST100 | 64.2 | 75.7 |
| | 11_EMGMST500 | 64.6 | 75.8 |

### Analytical ultracentrifugation (AUC) analysis of aggregates in anti-EPO mAb

The analytical ultracentrifugation sedimentation velocity (AUC-SV) method was used to determine the size distribution of the anti EPO mAb samples. A major advantage of AUC-SV is that the analyses are performed in the actual formulation buffer and there is no concern about loss of aggregates to a column matrix or filters. Therefore AUC-SV can also detect weakly associated and reversible high molecular weight species (HMWS). AUC-SV can characterize dimers and higher order aggregates, both covalent and non-covalent, determine their size, and quantitate the aggregate content.

AUC-SV is an inherently high variability technique. Standard AUC-SV analysis was performed at a protein concentration of 0.5 mg/mL in triplicate. Part of the anti EPO mAb samples were available only at 0.1 mg/mL, analysis at concentration lower than 0.5 mg/mL results in higher variability than usual. Samples formulated at 0.5 mg/mL were available only in small amounts and therefore the analyses were performed in one replicate only. Leftovers after Biacore analysis were available only for three of 0.5 mg/mL samples, and these were analyzed in duplicate. Also, only Glu samples at T=12wks were available.

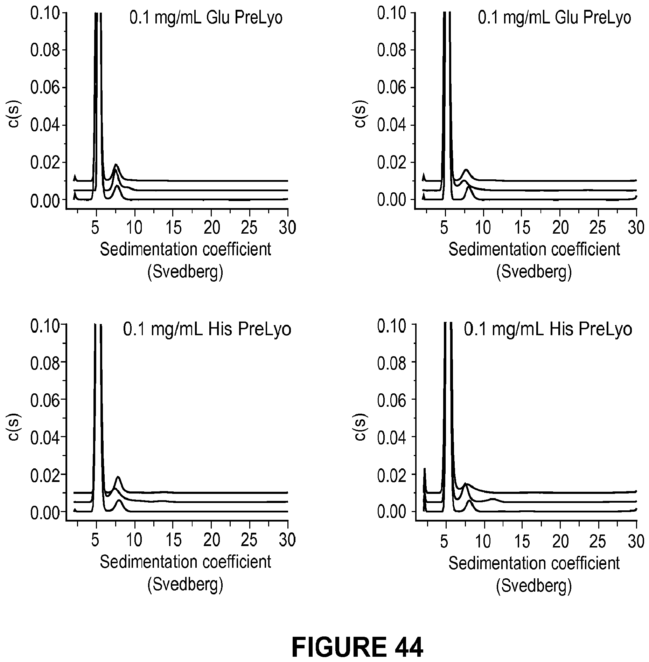

The high-resolution sedimentation coefficient distribution c(s) of the anti EPO mAb samples are illustrated in Figure 44. The vertical axis of the graph shows the concentration distribution and the horizontal axis shows the separation of the species on the basis of their sedimentation coefficients. The graphs are at 10-fold expansion for a better visualization of the small fractions of the higher molecular weight species. All the analyzed samples had very similar distribution pattern: the major HMWS detected in all replicate measurements was dimer, and small amounts of trimer was detected in some replicates. The size distribution patterns after T=7wks and T=12wks (not presented) were similar to those T=0wk presented in Figure 13. Traces of LMWS species were detected in most replicates.

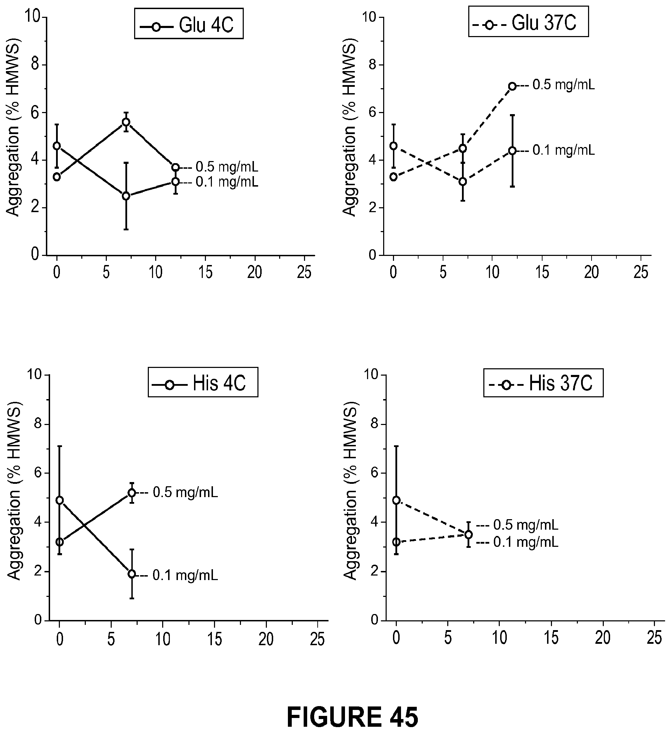

All the results of the AUC-SV analyses are summarized in Table 5 and for easier interpretation also graphically presented in Figure 45. As it can be seen from the data the HMWS content in all samples vary within 2-5 % and due to quite high variability of the method (the reasons discussed before) no conclusion on the effect of buffer composition (Glu vs. His) or protein concentration (0.1 vs. 0.5 mg/mL) can be drawn. The only observable effect was the increase of LMWS content in His buffer sample: PreLyo sample contained traces of LMWS below 0.1 % and those increased in PostLyo sample to 0.7 ± 0.4 %. There is also indication of increase HMWS content in Glu buffer at 37 °C after 12 weeks (7.1 %) but unfortunately corresponding His - 37 °C - 12wks sample was not available. A recommendation from this experimental set would be to perform much longer experiment (at least several times longer than this one) at elevated temperature (37 °C) to observe possible effects of buffer composition and protein concentration on long-term stability.

In summary, no detectable effect of buffer composition (Glu vs. His) or protein concentration (0.1 vs. 0.5 mg/mL) during 7 weeks experiment was observed by AUC-SV.

**Table 5. Summary of AUC-SV analyses of anti EPO mAb samples.**

| Time | Sample | Sample Code | LMWS | Monomer | HMWS % |
|---|---|---|---|---|---|
| T=0wks | Pre-Lyo | 03 EMGMST100 | 0.2 ± 0.2 | 95.2 ± 0.9 | 4.6 ± 0.9 |
| | | 04 EMHMST100 | 0.0 ± 0.1 | 95.1± 2.2 | 4.9 ± 2.2 |
| | | 05 EMGMST500 | 0.0 | 96.7 | 3.3 |
| | | 06 EMHMST500 | 0.0 | 96.8 | 3.2 |
| | Post-Lyo | 03 EMGMST100 | 0.1 ± 0.1 | 95.8 ± 0.6 | 4.1 ± 0.6 |
| | | 04 EMHMST100 | 0.7 ± 0.4 | 94.4 + 1.3 | 4.9 ± 1.6 |
| | | 05 EMGMST500 | 0.0 | 96.3 | 3.7 |
| | | 06 EMHMST500 | 0.0 | 95.5 | 4.5 |
| T=7wks | 4 °C | 03 EMGMST100 | 0.0 | 97.5 ± 1.4 | 2.5 ± 1.4 |
| | | 04 EMHMST100 | 0.0 | 98.1 ± 1.0 | 1.9 ± 1.0 |
| | | 05 EMGMST500 | 0.2 ± 0.1 | 94.2 ± 0.4 | 5.6 ± 0.4 (average of 2 rep.) |
| | | 06 EMHMST500 | 0.5 ± 0.4 | 94.3 ± 0.4 | 5.2 ± 0.4 (average of 2 rep.) |
| | 37 °C | 09 EMGMST100 | 0.0 | 96.9 | 3.1 ± 0.8 |
| | | 10 EMHMST100 | 0.0 | 96.5 | 3.5 ± 0.5 |
| | | 11 EMGMST500 | 0.1 ± 0.1 | 95.4 ± 0.5 | 4.5 ± 0.6 (average of 2 rep.) |
| | | 12 EMHMST500 | 0.2 | 96.3 | 3.5 |
| T=12wks | 4 °C | 03 EMGMST100 | 0.3 ± 0.2 | 96.6 ± 0.5 | 3.1 ± 0.5 |
| | | not analyzed | n/a | n/a | n/a |
| | | 05 EMGMST500 | 0.2 | 96.1 | 3.7 |
| | | not analyzed | n/a | n/a | n/a |
| | 37 °C | 09 EMGMST100 | 0.5 ± 0.1 | 95.1 ± 1.5 | 4.4 ± 1.5 |
| | | not analyzed | n/a | n/a | n/a |
| | | 11_EMGMST500 | 0.0 | 92.9 | 7.1 |
| | | not analyzed | n/a | n/a | n/a |

### Conclusion

In conclusion, the two lyophilized formulations maintained the antibody stability for at least 12 weeks of storage at 4°C and 37°C using SEC, AUC, reduced CE-SDS and DSC.

## Claims

1. A solid protein formulation comprising a stabilizer, a sugar alcohol, a sugar and a surfactant,
wherein
said stabilizer is histidine;
said sugar alcohol is mannitol;
said sugar is sucrose; and
said surfactant is polysorbate-80;
and wherein prior to being solidified, the concentration of histidine is 10 mM, the concentration of mannitol is 2%, the concentration of sucrose is 0.5% and the concentration of polysorbate-80 is 0.005% (w/v).

2. The formulation of claim 1, wherein the pH is between 6.0 and 8.0 prior to being solidified.

3. The formulation of claim 2, wherein the pH is 7.0 prior to being solidified.

4. The formulation of any one of the preceding claims, wherein the concentration of protein is between 0.1 mg/ml and 100 mg/ml prior to being solidified.

5. The formulation of claim 4, wherein the concentration of protein is 2 mg/ml prior to being solidified.

6. The formulation of any one of the preceding claims, wherein said protein is a glycoprotein.

7. The formulation of claim 6, wherein said protein is darbepoetin alfa.

8. The formulation of claim 6, wherein said protein is an antibody.

9. The formulation of claim 8, wherein said antibody is a monoclonal antibody or a polyclonal antibody.

10. The formulation of any one of the preceding claims, wherein said formulation is lyophilized.

11. A formulation comprising the formulation of claim 10, which is reconstituted with a liquid.

12. The formulation of any one of the preceding claims, wherein said protein is stable; optionally wherein said protein is stable for at least 1 month, at least 3 months, at least 6 months, at least 12 months, at least 18 months or at least 24 months, or optionally wherein said protein is stable at between 4 and 45 degrees C, or wherein said protein is stable at 4 degrees C, at 29 degrees C, at 37 degrees C or at 45 degrees C.

13. The formulation of any one of the preceding claims, wherein the percent oxidation of said protein is between 5% and 10%, or wherein the percent oxidation of said protein is less than 10%, or wherein the percent oxidation of said protein is less than 5%.

14. A method of preparing the solid protein formulation of any one of the preceding claims, said method comprising:
a) diluting said protein with a lyophilization buffer; and
b) lyophilizing said diluted protein.

15. A kit comprising the solid protein formulation of any one of the preceding claims and a reconstitution buffer.

## Patentansprüche

1. Feste Proteinformulierung, die einen Stabilisator, einen Zuckeralkohol, einen Zucker und
ein Tensid umfasst, wobei
der Stabilisator Histidin ist;
der Zuckeralkohol Mannitol ist;
der Zucker Saccharose ist; und
das Tensid Polysorbat-80 ist;
und wobei vor dem Verfestigen die Konzentration von Histidin 10 mM beträgt, die Konzentration von Mannitol 2 % beträgt, die Konzentration von Saccharose 0,5 % beträgt und die Konzentration von Polysorbat-80 0,005 % (w/v) beträgt.

2. Formulierung nach Anspruch 1, wobei der pH-Wert vor dem Verfestigen zwischen 6,0 und 8,0 liegt.

3. Formulierung nach Anspruch 2, wobei der pH-Wert vor dem Verfestigen 7,0 beträgt.

4. Formulierung nach einem der vorherigen Ansprüche, wobei die Proteinkonzentration vor dem Verfestigen zwischen 0,1 mg/ml und 100 mg/ml liegt.

5. Formulierung nach Anspruch 4, wobei die Proteinkonzentration vor dem Verfestigen 2 mg/ml beträgt.

6. Formulierung nach einem der vorherigen Ansprüche, wobei das Protein ein Glykoprotein ist.

7. Formulierung nach Anspruch 6, wobei das Protein Darbepoetin alpha ist.

8. Formulierung nach Anspruch 6, wobei das Protein ein Antikörper ist.

9. Formulierung nach Anspruch 8, wobei der Antikörper ein monoklonaler Antikörper oder ein polyklonaler Antikörper ist.

10. Formulierung nach einem der vorherigen Ansprüche, wobei die Formulierung lyophilisiert ist.

11. Formulierung, welche die Formulierung nach Anspruch 10, die mit einer Flüssigkeit rekonstituiert ist, umfasst.

12. Formulierung nach einem der vorherigen Ansprüche, wobei das Protein stabil ist; wobei das Protein wahlweise über mindestens 1 Monat, mindestens 3 Monate, mindestens 6 Monate, mindestens 12 Monate, mindestens 18 Monate oder mindestens 24 Monate stabil ist,
oder wobei das Protein wahlweise zwischen 4 und 45 Grad C stabil ist, oder wobei das Protein bei 4 Grad C, bei 29 Grad C, bei 37 Grad C oder bei 45 Grad C stabil ist.

13. Formulierung nach einem der vorherigen Ansprüche, wobei die prozentuale Oxidation des Proteins zwischen 5 % und 10 % liegt, oder wobei die prozentuale Oxidation des Proteins weniger als 10 % beträgt, oder wobei die prozentuale Oxidation des Proteins weniger als 5 % beträgt.

14. Verfahren zur Herstellung der festen Proteinformulierung nach einem der vorherigen Ansprüche, wobei das Verfahren Folgendes umfasst:
a) Verdünnen des Proteins mit einem Lyophilisierungspuffer; und
b) Lyophilisieren des verdünnten Proteins.

15. Kit, das die feste Proteinformulierung nach einem der vorherigen Ansprüche und einen Rekonstitutionspuffer umfasst.

## Revendications

1. Formulation de protéine solide comprenant un stabilisant, un alcool de sucre, un sucre et
un tensioactif, dans laquelle
ledit stabilisant est l'histidine ;
ledit alcool de sucre est le mannitol ;
ledit sucre est le sucrose ; et
ledit tensioactif est un polysorbate-80 ;
et dans laquelle avant la solidification, la concentration en histidine est de 10 mM, la concentration en mannitol est de 2 %, la concentration en sucrose est de 0,5 %, et la concentration en polysorbate-80 est de 0,005% (p/v).

2. Formulation selon la revendication 1, dans laquelle le pH est compris entre 6,0 et 8,0 avant la solidification.

3. Formulation selon la revendication 2, dans laquelle le pH est de 7,0 avant la solidification.

4. Formulation selon l'une quelconque des revendications précédentes, dans laquelle la concentration en protéine est comprise entre 0,1 mg/ml et 100 mg/ml avant la solidification.

5. Formulation selon la revendication 4, dans laquelle la concentration en protéine est de 2 mg/ml avant la solidification.

6. Formulation selon l'une quelconque des revendications précédentes, dans laquelle ladite protéine est une glycoprotéine.

7. Formulation selon la revendication 6, dans laquelle ladite protéine est la darbépoétine alfa.

8. Formulation selon la revendication 6, dans laquelle ladite protéine est un anticorps.

9. Formulation selon la revendication 8, dans laquelle ledit anticorps est un anticorps monoclonal ou un anticorps polyclonal.

10. Formulation selon l'une quelconque des revendications précédentes, dans laquelle ladite formulation est lyophilisée.

11. Formulation comprenant la formulation selon la revendication 10, qui est reconstituée avec un liquide.

12. Formulation selon l'une quelconque des revendications précédentes, dans laquelle ladite protéine est stable ; facultativement dans laquelle ladite protéine est stable pendant au moins un mois, au moins 3 mois, au moins 6 mois, au moins 12 mois, au moins 18 mois ou au moins 24 mois,
ou éventuellement dans laquelle ladite protéine est stable entre 4 et 45 °C, ou dans laquelle ladite protéine est stable à 4 °C, à 29 °C, à 37 °C ou à 45 °C.

13. Formulation selon l'une quelconque des revendications précédentes dans laquelle le pourcentage d'oxydation de ladite protéine est compris entre 5 % et 10 %, ou dans laquelle le pourcentage d'oxydation de ladite protéine est inférieur à 10 %, ou dans laquelle le pourcentage d'oxydation de ladite protéine est inférieur à 5 %.

14. Procédé de préparation de la formulation de protéine solide selon l'une quelconque des revendications précédentes, ledit procédé comprenant :
a) la dilution de ladite protéine avec un tampon de lyophilisation ; et
b) la lyophilisation de ladite protéine diluée.

15. Kit comprenant la formulation de protéine solide selon l'une quelconque des revendications précédentes et un tampon de reconstitution.
